# EUROPEAN PATENT SPECIFICATION

(11) **EP 3 781 192 B1**
(45) Date of publication and mention of the grant of the patent: **03.06.2026**
(21) Application number: 19719456.6
(22) Date of filing: 16.04.2019
(51) Int. Cl.: A61K 38/00, A61K 47/26, A61K 39/12, A61K 39/00, A61K 9/00, A61K 39/07, A61K 39/08

(54) **ADDITIVES FOR PROTEIN FORMULATIONS TO IMPROVE THERMAL STABILITY**
ZUSÄTZE FÜR PROTEINFORMULIERUNGEN ZUR VERBESSERUNG DER THERMISCHEN STABILITÄT
ADDITIFS POUR FORMULATIONS PROTÉIQUES EN VUE D'AMÉLIORER LA STABILITÉ THERMIQUE

(30) Priority: 16.04.2018 EP 18167611
(43) Date of publication of application: 24.02.2021
(73) Proprietor: Merck Patent GmbH, 64293 Darmstadt (DE)
(72) Inventor: ROSENKRANZ, Tobias, 64283 Darmstadt (DE); SCHACHE, Janet, 64404 Bickenbach (DE)
(74) Representative: Merck Patent Association
(86) International application number: PCT/EP2019/059755
(87) International publication number: WO 2019/201894

(56) References cited:
- WO-A1-2011/014418
- WO-A2-2010/132508
- US-A1- 2013 209 503
- US-A1- 2013 216 532
- US-A1- 2015 079 130
- US-A1- 2017 121 414
- PASSOT S ET AL: "Physical characterisation of formulations for the development of two stable freeze-dried proteins during both dried and liquid storage", EUROPEAN JOURNAL OF PHARMACEUTICS AND BIOPHARMACEUTICS, ELSEVIER SCIENCE PUBLISHERS B.V., AMSTERDAM, NL, vol. 60, no. 3, 13 May 2005 (2005-05-13), pages 335 - 348, XP027805066, ISSN: 0939-6411, [retrieved on 20050801]
- CAPELLE ET AL: "High throughput screening of protein formulation stability: Practical considerations", EUROPEAN JOURNAL OF PHARMACEUTICS AND BIOPHARMACEUTICS, ELSEVIER SCIENCE PUBLISHERS B.V., AMSTERDAM, NL, vol. 65, no. 2, 5 January 2007 (2007-01-05), pages 131 - 148, XP005823137, ISSN: 0939-6411, DOI: 10.1016/J.EJPB.2006.09.009

## Description

The present invention relates to protein formulations comprising maltose, which are suitable to improve the thermal stability against denaturation and deactivation. In particular, the present invention relates to methods for preparation of formulations maltose for thermostabilizing vaccine formulations.

### State of the Art

Proteins or peptides are commercially used in a variety of pharmaceutical forms, for example vaccines. Many of these comprise proteins and structures/complexes that are based on proteins. The said proteins are often produced by recombinant DNA technology or other means. Proteins utilized in vaccine formulations can be of bacterial or viral origin. Said proteins can be classified into groups including, but not limited to, toxins, toxoids or mutated proteins. Furthermore, vaccines can be derived from said bacterial or viral proteins/peptides for example in the form of epitope vaccines, chemically inactivated toxins (toxoids), glycoproteins, or conjugated proteins, where a pharmaceutically active substance is covalently bound to a carrier protein. These entities could include protein-protein conjugate (e.g. Tetanus toxoid), a fusion-protein, a protein-polysaccharide conjugate, a protein-nucleic acid conjugate, a protein-DNA conjugate, a protein-RNA conjugate, a protein-small molecule conjugate, a protein-dye conjugate, a protein-alum adsorbate, a luminescent or bioluminescent protein, a biotinylated protein. Additionally, proteins form one of the building blocks of viral fragments.

From chemical view point proteins are large organic molecules or macromolecules made up of amino acid residues covalently linked together by peptide bonds into a linear, unbranched polypeptide chain with relative molecular mass ranging from a few thousand to millions Da. The useful properties of proteins as drugs depend upon the polypeptide chain adopting a unique three-dimensional folded conformation, that is, the tertiary structure of the protein. It is this unique folding that is responsible for the protein being highly selective in the molecules it will recognize. However, the ability to maintain a unique three-dimensional structure is precisely one of the obstacles that makes the use of polypeptides in human and animal diseases fraught with problems.

Therefore, maintaining the potency of vaccine or protein particles against degradation is a major challenge in providing proper immunization services, because it is well known that biological materials, including biological materials in solution, are susceptible to inactivation due to heat, oxidizing reagents, salts, etc.

Virus particles, bacteria and other infective agents used in vaccines may be readily inactivated after a short period at ambient temperature, but low temperature storage is costly. Even short exposures to high temperatures lead to loss of vaccine activity even at low temperature limit vaccine use and raise costs thereby limiting vaccine distribution particularly in under developed third world, countries.

From literature it is known that up to 80 % of the costs of vaccination programs is due to the cold chain problem (for keeping the vaccines cold), which means, it is imperative to keep the vaccines cold and to maintain their efficacy.

Typically, this requires keeping vaccines refrigerated at all times from production to administration - a major undertaking especially in remote regions of developing countries.

Termed the cold chain problem, the high cost and risk associated with protecting vaccines from deterioration has been identified by the World Health Organization as one of the most important challenges for the extension of global vaccination programs. Therefore, there are various approaches to stabilize immunizing formulations and attempts have been made to solve this problem by the addition of low-cost, biocompatible additives and to slow down the degradation of virus particles. For example, it is proposed to improve the storage time of adenovirus type 5 by the addition of anionic gold nanoparticles (10⁻⁸-10⁻⁶ M) or polyethylene glycol (PEG, molecular weight ~8,000 Da, 10⁻⁷-10⁻⁴ M) to increase the half-life of a green fluorescent protein expressing adenovirus from ~48 h to 21 days at 37 °C (from 7 to >30 days at room temperature). A stabilizing effect is also known from sucrose, which is added at low concentrations. It is found that addition of PEG and sucrose can maintain immunogenicity *in vivo* for viruses stored for 10 days at 37 °C. But these stabilization effects are not sufficient to achieve storage stability at high temperatures of more than 60 °C. However, the latter is necessary for the safe use of vaccine formulations in tropical areas and it is surprising that, as such, liquid protein formulations, like vaccine formulations, are currently produced without any specific thermostabilizing agent or excipient that would result in thermostability to temperatures above 60°C. One reason for this is, among others, that the addition of additives to existing vaccines usually means to get again through the costly process of approval by health authorities, which is commercially unattractive. Therefore, it would be desirable to stabilize vaccine formulations against inactivation at elevated temperatures by the addition of additives or excipients, preferably by those already recognized as compatible ingredients for pharmaceutical compositions. As mentioned in a review of Pellicca, M. et al., one approach has been the addition of sucrose to vaccine formulations [Pelliccia, M. et al. Additives for vaccine storage to improve thermal stability of adenoviruses from hours to months. Nat. Commun. 7, 13520 doi: 10.1038/ncomms13520 (2016)]. Trehalose, glycerol, glucose, maltose and raffinose have also been suggested for this use, but the desired effect could only be found at high concentrations and not at temperatures higher than 40°C. The most effective thermal stabilizing additive PEG, as reported by Pellicca, M. et al., has proved to be unsuitable for vaccine formulations because up to 50% of humans show allergic reactions to it.

In WO 2012/125658 A1 a process for the production of a stabilized vaccine composition of immunogens, particularly labile immunogens, is provided wherein oral granules are aerosolized with an antigenic viral culture at a temperature of about 25 ° C to about 50 °C, preferably at about 30 °C, such that immunogen coats are dried onto the particles. The vaccine product containing particles having a moisture content between about 0.1% w/w and 10% w/w so as to give a stabilized vaccine composition.

In US 2013/209503 A1 freeze-dried and lyophilized vaccine composition is stabilized by providing first a polysaccharide-protein conjugate, which is stabilized by combinations of an amorphous excipient selected from the group consisting of sucrose, lactose, trehalose, dextrose, xylose, cellobiose, raffinose, isomaltose and cyclodextrins and a buffer selected from salt of a group consisting of citrate, histidine, phosphate, tris, succinate, and acetate buffers.

In WO2011/014418, a formulation capable of enhancing thermostability and shelf-life of a biological product comprising a tertiary amine N-oxide is disclosed.

US2013/0216532 discloses highly concentrated, stable pharmaceutical formulations of a pharmaceutically active anti-HER2 antibody comprising a buffering agent, a stabilizer or a mixture of two ore more stabilizing agents, a nonionic surfactant and an effective amount of at least one hyaluronidase enzyme.

WO2010/132508 relates to a matrix for substantially dry storage of a biological sample comprising a borate composition and a zwitterionic stabilizer.

PASSOT S ET AL (European Journal Of Pharmaceutics And Biopharmaceutics, Elsevier Science Publishers B.V., vol. 60, no. 3, 13 May 2005, pages 335-348) discloses the influence of 13 combinations of excipients on two stable freeze-dried proteins during storage at ambient temperature.

CAPELLE ET AL (European Journal Of Pharmaceutics And Biopharmaceutics, Elsevier Science Publishers B.V., vol. 65, no. 2, 5 January 2007, pages 131-148) discusses the chemical and physical degradation pathways of proteins and the most important HTS techniques and their applicability in protein formulation.

US 2017/121414 relates to a pharmaceutical composition comprising an anti-CD38 antibody and a hyaluronidase.

US 2015/079130 relates to adjuvant compositions and production methods for the same that enhance the immune response in a recipient, suitable for oral administration.

In addition, there is a whole series of publications and patent literature which attempts to solve the stability problem of corresponding proteinaceous formulations at higher temperatures in a similar manner, but at the cost that injectable solutions prior to application need to be re-established. The latter however would be a significant problem in underdeveloped countries and in crisis areas. Here, in connection with elevated ambient temperatures, even an improvement in the stability of the protein formulations for several hours would represent an improvement.

### Object of the invention

Thermostability and cold-chain interruptions are major obstacles in assuring delivery of stable vaccine doses especially to remote areas in poverty stricken countries. Currently vaccines are refrigerated during shipping, however 25% of all liquid and 50% of all lyophilized vaccine doses shipped to developing countries are lost because continuous refrigeration cannot be guaranteed.

A major object of the present invention is to provide thermally stabilized vaccine formulations which can be made available preferably with the same quality as comparable products and which are stable for a long time when stored at temperatures up to and above 60°C.

### Short summary and subject of the invention

Maltose in a range of 0.5 to 1.8 M is added to a protein or peptide formulated in an aqueous buffer in an optimized concentration. The melting temperature Tₘ of the protein or peptide is thereby substantially elevated, so that high temperatures up to and above 60°C can be tolerated.

The present invention delivers stabilized solutions of proteins, advantageously in relatively high concentration of about 2,0 mg/ml, comprising at least maltose as stabilizing agent, which minimizes the degradation and inactivation of the comprising proteins, which directly has positive impacts on maintaining their efficacy and allows that these formulations of the present invention to be stored and even transported to underdeveloped markets in tropical zones.

More particularly, the present invention relates to a method for thermo-stabilization of liquid protein formulations as characterized in claim 1 and in the particular embodiments according to claims 2 to 11.

### Detailed description of the invention and preferred embodiments

The present invention relates to a method of obtaining liquid protein or peptide formulations, which are thermo-stabilized and if desired, in a direct usable formulation, comprising proteins which may be produced by recombinant DNA technologies or other means, protein-conjugates i. e. protein-saccharides, protein-nucleotides, protein-RNA/DNA or protein-peptides, cross-linked proteins, like formaldehyde treated proteins like tetanus toxoid and ultimately larger structures or other proteins utilized in vaccine formulations, which can be of bacterial or viral origin, viral protein subunits, enzymes, hormones or others. Accordingly, in the following, when referring to protein formulations, it is always meant also peptide formulations, unless it is clearly spoken of specific formulations containing one or the other.

This invention also relates to the stabilized formulations wherein the proteins keep their folded, effective structure, even when the temperature rises to values of up to 60 ° C. The present invention also relates to such formulations, which keep their stabilization for up to three or even four days. Advantageously, the stabilized protein formulations can be readily obtained by adding to the solutions, optionally buffered and having their pH adjusted to an optimum range for the protein contained, maltose in a concentration in the range of 0.5 - 1.8 M. The formulation may also contain a reducing agent. Liquid formulations are prepared according to the method of the invention comprising the proteins in a concentration in the range of between 0.05 to 1.8 mg/ml. The thermostabilizing effect of maltose is evidenced by the increase in the melting temperature of the contained protein; and at the same time the increase of the protein melting temperature is an indicator of increased thermal stability of the protein in the formulation. By specific experiments it was found that, on the one hand, depending on the type of protein and the added excipient, different amounts of maltose have to be added in order to achieve the desired stabilization of the contained protein, while it is also advantageous, if the pH value is adjusted in the stabilizing solution depending on the protein contained. Concentrations in the range from .5 - 1.8 M are useful in the protein solutions for the desired protein stabilization to minimize degradation and inactivation during storage as well as during exposure to temperatures higher than room temperature, especially to temperatures higher than 40 °C. As previously stated, the reliable stabilization of a protein also depends in particular on the pH of the solution. Depending on the protein, the optimum pH can be in the range between pH 6 to 8 and is advantageously set with a suitable buffer.

In this way, protein solutions can be stabilized against a deactivating influence of elevated temperatures. To improve the thermostability, only those experiments were considered in which at least an increase of the melting temperature of the protein by 5 ° C could be determined by the addition of maltose.

For further use, the thus stabilized formulations can be filled in bottles for further dosing, dilution or processing. However, they can also be formulated as immunization solutions which optionally can be finished as solutions for use in pharmaceutical injection vials, unless further processing or dilution is required. Furthermore, in another form of application so stabilized protein solutions can be presented in an immunoassay test kit or in preparations and bottlings for analysis. Also, stock formulations can be stabilized in that manner, which are shipped globally for subsequent fill finish.

The protein comprising liquid formulations prepared according to the claimed method may comprise in addition to maltose one or more additional compounds, such as amino acids, proteins, chelating agent, buffers, salts, preservatives, stabilizers, antioxidants, emulsifiers, plasticizer or lubricants.

A peptide is an organic-chemical compound that contains peptide bonds between amino acids. According to their number, oligopeptides are distinguished with few of polypeptides having many amino acids. Long polypeptide chains are also referred to as proteins, in particular those formed by protein biosynthesis. Due to this chemical relationship of peptides and proteins, both classes of substances are meant, if in the following of proteins is spoken.

In literature the expression "thermostability" is frequently used, but often in a misleading manner. In literature, this usually only means that a protein solution can be stored at slightly elevated temperatures below 40 ° C. By increasing the storage temperature to 40°C, which is still well below the unfolding-transition mid-point (Tₘ) of many proteins (e.g. Anthrax PA Tₘ = 48°C) the spontaneous unfolding rate of a protein is elevated. In terms of equilibrium thermodynamics, there is a certain probability that a protein may unfold spontaneously at any point in time, however, the likelihood of such an unfolding event is temperature dependent. By elevating the storage temperature, the frequency of a potential unfolding event is increased, and therefore the long-term storage of a protein formulation can be assessed in a reduced period of time.

Here, according to the present invention, the expression "thermostability" is used in the context that a protein or peptide mostly stays in its native, not unfolded form during storage at temperatures higher than 50 °C and up to 60 °C.

The present analytical efforts are directed to thermostability by determining the thermodynamic transition mid-points of thermally stressed proteins. To this end, the effect of model molecules or of selected excipients is examined for their stabilizing effect on the structure of model proteins at high temperatures, this means at temperatures higher than 50 °C and up to 60 ° C. At the same time, the experiments analyze in which concentration ranges the thermostabilizing excipients are effective.

By definition, an excipient eliciting a dTₘ of >5°C is a good stabilizer. If dTₘ is <5°C and >0°C the excipient is defined as a poor stabilizer. A negative dTₘ identifies a molecule as a protein destabilizer.

The transition mid-point Tₘ is determined by fluorescence spectroscopy, this means by Nano-Differential Scanning Fluorimetry (nanoDSF) using a protein solution comprising the protein in a concentration of 0.05-1,8 mg/ml.

The prepared protein solution is heated with a heating rate of 1°C/minute and the ratio of the fluorescence emission at 350/330 nm is recorded and plotted vs. temperature. Typically, the resulting curve resembles a Boltzmann function and the inflection point of this curve represents the mean unfolding temperature Tₘ. From this curve the change in Tₘ determined by subtracting T_{m native} from Tₘ excipient formulation, which yields to dTₘ.

The expression "fraction folded" is used in the following and represents the amount of native protein in the liquid formulation. Secondly, the expression "thermal denaturation midpoint" is also used in connection with stability of a protein, and it is defined, that a deviation of 10% in either direction as acceptable. It is important to note that any change in transition mid-point, also to higher temperatures, is a result of either a change of the protein conformation or of the data quality. The latter can for example be affected by aggregation of proteins or by numerous of other effects. Therefore, transition temperatures are required that deviate less than 10% from the initially measured value.

As used herein, the term "sugar" refers to any of a group of water-soluble carbohydrates of relatively low molecular weight. The term sugar includes reducing sugars (such as maltose), non-reducing sugars (such as sucrose), sugar alcohols (such as sorbitol) and sugar acids (such as lactobionic acid).

All ranges set forth herein are intended to be inclusive the lower and upper limit of the range. The term "about" is meant to encompass given ranges a little bit. Throughout this application, the term "about" is used to indicate that a value includes the standard deviation of error for the method being employed to determine a value.

The use of the word "a" or "an" when used in conjunction with the term "comprising" in the claims and/or specification may mean "one", but it is also consistent with the meaning of "one or more", "at least one", and "one or more than one".

As used in this specification and claim(s), the words "comprising" ( and any form of "comprising", such as "comprise" and "comprises"), "having" (and any form of "having", such as "have" and "has"), "including" (and any form of "including", such as "includes" and "include") or "containing" ( and any form of "containing", such as "contains" and "contain") are inclusive or open-ended and do not exclude additional, unrecited elements or method steps.

It is also specifically understood, that any numerical value recited herein includes all values from the lower value to the upper value, i. e., all possible combinations of numerical values between the lowest value and the highest value enumerated are to be considered to be expressly stated in this application. For example, if the range is stated as 1 % to 20 %, it is intended that values such as 2% to 5%, 10%, to 15%, or 3% to 4,5%, etc., are expressly enumerated in this specification.

"Contacting" refers to the process of bringing into contact of at least two distinct species such that they can interact or react.

"Excipients" generally refer to compounds or materials that are added to ensure or increase the stability of the therapeutic agent, i. e., for long term stability.

A "stable" formulation or composition is one in which the biologically active material or protein therein essentially retains its physical stability and/or chemical stability and/or biological activity on storage. Stability can be measured at a selected temperature for a selected time period. Trend analysis can be used to estimate an expected shelf life before a material has actually been in storage for that time period.

"Pharmaceutically acceptable" refers to those active agents, salts, and excipients which are, within the scope of sound medical judgement, suitable for use in contact with tissues of humans and lower animals without undue toxicity, irritation, allergic response and the like, commensurate with a reasonable benefit/risk ration, and effective for their intended use.

As already mentioned above, protein solutions of the present invention are adjusted to a specific pH. In order to keep the pH stable, suitable buffers are added to the protein solutions.

The buffers can be added in form of a carrier fluid suitable for dissolving and/or dispersing the protein to be solved. The buffer is usually selected from a pharmaceutically accepted buffer system. The preferred buffer is a pharmaceutically accepted buffer system with the ability to resist a change in pH upon addition of acid, base, inorganic compound, organic compound or solvent or diluent. Buffering components such as phosphate or citrate are included to control the pH of the vaccine containing solution, as well as to adjust the solution osmolarity. The buffer concentration may range from 5 mM to 2 M, with the pH of the solution adjusted to a range from about pH 4 to about pH 10, preferably to a range from about pH 6 to about pH 8.

A pharmaceutically acceptable buffer may be selected from the group consisting of potassium phosphate, sodium phosphate, sodium acetate, histidine, imidazole, sodium citrate, sodium succinate, HEPES, Tris, Bis-Tris, ammonium bicarbonate, and other carbonates. The buffer may comprise a pH ranging from about pH 4 to about pH 10, preferably from about pH 6 to pH 8, but also from about pH 6 to about pH 7.

The amount of maltose in the aqueous thermo-stabilized solution may range from very low concentrations to quite high concentrations. Thus, for stabilization, maltose can be added in rather small amounts, but often there is a particularly effective thermostabilization of the proteins if the sugars contained are at rather high concentrations, namely, when the addition to the solution is in amounts of several moles. It has thus been found by the experiments that particularly good stabilization results can be achieved if maltose is added in amounts of from 0.5 to 1.8 M protein solution.

The aqueous solution can further comprise pharmaceutically acceptable surfactants, polymers, amino acids, and other pharmaceutically acceptable excipients. Polymers can be included to stabilize the protein. These polymers can be added in amounts of 0,1 % w/v and more. Surfactants can be added to decrease surface tension and to displace protein molecules from the surface. Surfactants may also increase the solubility of other formulation components. Surfactants may be comprised in amounts of 0,005 % and more by weight of said protein containing formulation.

Divalent cations and amino acids can be included to stabilize the protein and to adjust the pH and osmolarity of the solution. The concentration of the divalent cation may range from 0,1 mM to about 100 mM and the amino acid may be contained in a concentration in the range from about 0,1 % to about 1% (w/v).

In sum, this means that depending on what the protein or peptide solution according to the invention is to be used and depending on the properties of the proteins or peptides contained in the solution, different components of the final formulation can be added.

According to the present invention, the thermostabilizing excipient is Maltose Maltose is added in various concentrations to the protein formulations. The protein concentration is within 0.05 and 1.8 mg/ml. An increased thermostability can be detected in a regime of 0.5- 1.8 M.

The aqueous protein solutions of the present invention can be provided in a primary container such as in a vial, either made of glass or plastic/resin, or as a foil pouch device or any other suitable and compatible device. Depending on the desired subsequent use, the solutions provided in this way can be present in amounts of a few µl up to many liter amounts.

The present invention provides a method whereby proteins or peptides used in pharmacy and medicine, which are in solution, can be protected against a destabilizing and inactivating influence of elevated temperatures, meaning the proteins can be thermostabilized. In particular, this stabilizing effect relates to protection against the onset of protein unfolding and agglomeration, which generally results in inactivation and loss of protein activity/or efficacy of the drug. The method of the present invention is applicable to any protein or to vaccines comprising viral protein subunits, which can be used at microgram quantities. The proteins can be proteins as components of vaccines, but also enzymes, hormones or other therapeutically or analytically used proteins, like enzymes, antibodies, nanobodies or monobodies. Depending on the protein type and on its application, the solutions are offered in different amounts, packaging or devices, but also in combination with other required for the desired application compositions. Accordingly, they may be part of a bioassay or may already be present as a solution in an application or analysis or diagnostic kit, or in enzyme-containing molecular biology kits. This means, said proteins can be included in a vaccine as well as in diagnostic assay for the detection of an agent in a sample of a subject of having a disease. The agent of disease can be from an infection, a tumor, or an allergen or other.

As described above, the present invention relates to the thermostabilization of corresponding protein formulations. Through a planned experimental program, the influence of selected additives on the thermostability was investigated using sample proteins as enumerated above.

In order to test as different proteins as possible for the thermostabilization by the addition of suitable excipients as listed above, the experiments were carried out using the following sample proteins:
enzymes, especially lactate dehydrogenase, and lysozyme as model enzymes,
   and
vaccine proteins, especially AnthraxPA (Anthrax protective antigen), Tetanus toxoid, and Diphtheria toxoid (CRM197) as model vaccines.

In order to demonstrate an effective thermostabilization by addition of one or more of the excipients, a liquid formulation is prepared comprising said proteins in a concentration in the range of between 0.05 to 1.8 mg/ml. To do so a stock solution of the protein is prepared. Also, an excipient stock solution is prepared in the applicable buffer. Protein, excipient and, if required, buffer are combined to yield the formulations described in the examples. As shown in chapter "Examples" melting temperatures of these proteins (Tₘ) in the presence of most efficient and most applicable thermostabilizers are tested, because the increase of the protein melting temperature is an indicator of increased thermal stability of the protein in liquid formulations.

The determination of the denaturation temperature or melting temperature Tₘ can be carried out in a high throughput fashion using differential scanning fluorimetry on small volumes (NanoDSF: Prometheus.NT48 equipped with standard capillaries, NanoTemper Technologies GmbH). Additionally, for some formulations Tm was determined using circular dichroism spectroscopy to gain additional structural information about proteins in the said formulations (Spectropolarimeter J-815, Jasco Deutschland GmbH using a 0.1 cm quartz Suprasil cuvette type 110-QS from Hellma).

Proteins and peptides generally fold into a stable three-dimensional structure that is stabilized by a large number of weak interactions of residues within the polypeptide chain. By elevating the temperature of a formulation the increased thermal motion and other factors cause denaturation of a protein. To quantify the thermal stability of a protein a temperature called Tm is used.

Tₘ represents the temperature of an equilibrium transition midpoint herein obtained for the heat induced denaturation of the model proteins. Assuming a two-state transition from a native to a non-native state (unfolded/partially unfolded) the Tₘ represents a temperature at which both states are equally populated. Measurements of the transition midpoint are affected by the kinetic stability of a substance and therefore only such values obtained under equal conditions are comparable. All Tₘ values reported herein were recorded at a heating rate of 1 °C/min. A Tₘ value that is elevated upon addition of an excipient indicates the stabilization of a protein.

Denaturation of proteins can be measured by various spectroscopic techniques, which leverage on specific optical properties of a protein.

Differential scanning fluorimetry is a fluorescence based method, which leverages on the specific emission properties of the natural proteinogenic amino acid tryptophan. The fluorescence emission of the indole side chain is solvatochromic due to its two distinctive dipole axes. When imbedded into the interior of a protein, which is a more lipophilic environment, tryptophan typically shows a fluorescence emission maximum of around 328-330 nm. When a protein is denatured, the tryptophan residues become progressively less shielded from the surrounding water. Therefore, the emission maximum of tryptophan approaches 350 nm. This spectral shift to higher wavelength is called a red-shift. NanoDSF records this shift by recording the emission of a protein at 330 nm and 350 nm and plotting the ratio thereof vs. temperature. This typically results in a sigmoidal curve with a positive amplitude. Fluorescence spectroscopy probes mostly for tertiary structure transitions. It should be noted that the fluorescence spectrum of a protein represents an overlay of all emission spectra of the amino acids contained therein and therefore the emission properties of a specific protein may differ from the values stated above.

CD-spectroscopy leverages the differential absorption of circular polarized light by a chiral substance or a large molecule with a non-symmetrical structure such as proteins. Depending on the wavelength regime used, CD-spectroscopy can be used to probe the secondary or tertiary structure of a protein. Herein we use CD-spectroscopy exclusively to obtain information about the secondary structure of proteins. Natively folded proteins exhibit very distinctive CD-Spectra based on their structural composition. Upon heat induced unfolding, the differences in absorption of circularly polarized light reduces and the CD-Signal approaches zero. To determine Tₘ the CD-Signal at a specific wavelength, typically 222 nm, 218 nm, 208 nm or 196 nm, is plotted vs. temperature and subsequently fitted with a sigmoidal function.

Regardless of the method used, there are specific properties of the protein that report structural features of the protein. If upon addition of an excipient the transition midpoint is elevated, the added excipient stabilizes the protein. Additionally, the steepness of the sigmoidal curve can be modified by the addition of an excipient. The steepness of the curve reflects the kinetic stability of a protein. While Tₘ describes at which temperature a protein unfolds, the steepness of the unfolding curves indicates how fast the said transition occurs.

In the case of fluorescence spectroscopy an additional feature can alter the behavior of the sigmoidal curve. The said curve can show a negative amplitude indicating a shift of fluorescence to lower wavelength instead of the previously described red-shift. This can be explained by protein aggregation. If not all tryptophan residues are buried within the proteins the emission of the natively folded protein can be at wavelength greater than 330 nm. Upon unfolding tryptophan residues would get more exposed to water, however upon aggregation said residues are buried within the lipophilic core of the aggregate. In spite of the negative amplitude a sigmoidal fit can still be used to determine a temperature reflecting the thermal stability of a protein.

Now, in order to evaluate which additives or excipients are best suited for thermo-stabilization of proteins and in what amounts they have to be added to produce the desired effect, in the first step test formulations with AnthraxPA as a model vaccine were prepared and examined over three days. The detailed formulation compositions are summarized in Table 1 in chapter "Examples".

The aim of these experiments is to find the most suitable excipient or mixture of excipients for thermo-stabilization of protein compositions of different origin over several days.

Since to date stabilizing agents have to be used at high concentrations, such compositions cannot be used to prepare pre-filled syringes because patients would likely not tolerate the stabilizing agent. A dilution step is therefore required prior to administration to the patients. Another objective of these experiments is also to determine the highest likely tolerable levels of stabilizing agents for use in vitro assays. Based on the results of the experiments, the protein concentration can be adjusted and a vaccine stock solution can be generated, which can provide both heat protection for the solute protein but can still be effectively administered to the patient after dilution.

For example, it has been found that melibiose (embodiment not covered by the claimed invention) can most likely be tolerated by a patient up to a concentration of 250 mM. From previous screenings, however, it is known that at least 2.25 M of melibiose are required to stabilize AnthraxPA at high temperatures. Therefore, the protein concentration is increased by a factor of 2250 mM (stock) / 250 mM (final value) = 9. But for patient use it is required to dilute this stabilized vaccine stock formulation for at least the nine-fold amount.

In accordance with these considerations, test formulations are prepared to examine the model proteins enumerated above which excipients are best suited as a physiologically acceptable additive for the best possible thermal stabilization at temperatures higher than 40 °C, preferably higher than 60 °C. From available potential excipients, which are recognized as acceptable to the patient in liquid pharmaceutical formulations lactulose (embodiment not covered by the claimed invention) and maltose have been selected first as promising temperature stabilizing additives and corresponding protein formulations have been prepared with these additives and examined. TMAO, choline dihydrogen phosphates, and melibiose (embodimentS not covered by the claimed invention) were also tested under the same conditions and the results were compared with those of lactulose (embodiment not covered by the claimed invention) and maltose.

First, the samples are these samples are spiked with the selected excipients suitably subjected to fluorescence spectroscopy to verify that all protein samples are natively folded and then to determine if they are suitable, to stabilize the proteins at elevated temperatures including elevated protein levels (up to 1.8 mg / ml). Following these tests, incubation is carried out at specified temperatures, especially at 4 °C and in the range between 50 to 60 °C, and if appropriate, at a higher temperature.

The tests have shown that in particular, when melibiose is added as stabilizing additive to the protein formulations, in general better thermo-stabilization occurs and the formulations are generally stable at higher temperatures and have a higher melting point, as can be demonstrated by melting curves and by determination of the transition midpoint.

In order to verify the thermostabilizing effect of the various excipients over a longer period of time, after incubations periods of 6 hours, 24 hours, 32 hours, 48 hours and 72 hours, small samples are taken and the structural state and stability of the comprising protein is evaluated by means of fluorescence spectroscopy.

To determine the status whether the examined protein-containing samples are still native or damaged, two parameters are now monitored repeatedly by fluorescence spectroscopy during the duration of experiments. The first parameter refers to the amount of damaged or unfolded protein in the liquid formulation. The second parameter refers to the thermal denaturation midpoint. (However, it is essential for the quality and reliability of the determined data that a deviation of the determined transition temperature is less than 10%. This deviation may be caused by various effects, such as aggregation or other influences.)

Based on the experiments carried out, it has been found that a significant improvement in the temperature stability can be achieved for proteinaceous formulations by the addition of maltose. For example, in its native formulation AnthraxPA has an unfolding transition temperature of 48 °C, which would not permit storage at or above 50°C. Maltose is suitable to stabilize liquid AnthraxPA formulation at 55 °C for at least three days..

However, comparable results are not always found in all cases. For example, the evaluation of the thermostability of CRM197 in liquid formulation is somewhat different than that of AnthraxPA formulations. In its native formulation CRM197 shows a biphasic unfolding transition when assayed with fluorescence spectroscopy. The initial transition relates to a localized loss of structure while on a global scale the protein remains structurally intact. This is why for studying the stability of CRM197 different criteria have to be applied. In this case conditions for two modifications are required. First, the median unfolding temperature cannot be used as a readout, because a conformational scrambling occurs due to local loss of structure which cannot be stabilized by simply adding the selected excipients. In addition, changes in its initial transition may affect the determination of the second unfolding temperature leading to an erroneous assessment of the global structural state based thereon. Secondly, the extent of damage inflicted on the temperature-depending region of CRM is affecting the fluorescence emission ratio 350 nm to 330 nm. In order to evaluate the effect of the excipients tested here, all results are compared with results measured in formulations comprising the protein in its native formulation incubated and denatured at about 50 °C. Under the same conditions CRM197 shows an unfolding in its native formulation at a temperature of 53 °C. By addition of melibiose (embodiment not covered by the claimed invention) or maltose to corresponding CRM197 formulations an unfolding is largely suppressed for at least 72 hours when the formulation is incubated at 50 ° C. At this temperature, but also at 55 °C the addition of melibiose has a stabilizing effect even for 96 hours, while maltose stabilizes CRM197 only for 72 hours. Incubation at 60 °C reverses the ratio. The measured results show that CRM197 is stabilized by Maltose for 48 hours at 60°C, while Melibiose (embodiment not covered by the claimed invention) stabilizes CRM only for an incubation period of 24 hours at 60 °C..

By adding Maltose to protein formulations, formulations can be thermostabilized for three days in which the protein is contained in a concentration of up to 1,8 mg / ml. The proteins may be vaccine proteins. It may be proteins such as AnthraxPA (Anthrax Protective Antigen) or for example CRM197 or similar proteins. The proteins may be heterologically expressed proteins or overexpressed proteins.

The experiments identify the best stabilizations for liquid protein formulations containing AnthraxPA or CRM as proteins. The AnthraxPA formulation contains 5 mM HEPES as buffer and 50 mM NaCl.

As mentioned for the previous experiments and investigations with AnthraxPA and CRM197, formulations with the enzymes lactate dehydrogenase and lysozyme as model proteins are also tested for being thermostabilized. Excipients of Table 1 are added in suitable concentrations and tested for their possible thermostabilizing effect. In this context, further potential thermo-stabilizers are tested and selected from the group of ionic liquids (embodiment not covered by the claimed invention), which are four biodegradable compounds and which are selected and prepared based on compounds that can be regularly found in the human body: Choline Chloride is found in bile, while Glucose, Sucrose, Sorbitol, and Glycerol are natural metabolites in the respective sugar or fat pathways.

In this context, it has been found by the investigations carried out here for thermostabilizing of said vaccine and enzyme proteins that, depending on the protein contained in the solution, the concentration of added excipients must be adjusted to show an effect. It has been also found, that the same excipient can show different effects depending on its concentration and on the protein type.

Surprisingly, based on the results found, it cannot easily be assumed from the fact that a selected excipient belongs to a certain substance class that it is also a good thermostabilizing additive for a particular protein in solution because a related substance provides good results with another protein.

The summarized results of the investigations show that added excipients which inflicted the largest change in melting temperature of tested proteins, and which show the broadest applicability and which are suitable as stabilizer for all three vaccine proteins tested belong to very different substance groups. These excipients are TMAO (embodiment not covered by the claimed invention), Choline dihydrogenphosphate (embodiment not covered by the claimed invention), Melibiose (embodiment not covered by the claimed invention), Maltose and Lactulose (embodiment not covered by the claimed invention).
a) TMAO (embodiment not covered by the claimed invention) shows a thermostabilizing effect beginning at a concentration of 250 mM. Optimum stabilization results are obtained at a concentration in the range of 1 to 4 M in solution.
b) Choline dihydrogenphosphate (embodiment not covered by the claimed invention) shows a thermostabilizing effect starting with a 1 molar solution. Optimum results are found for compositions when the concentration is in the range of 2 to 4 M.
c) Melibiose (embodiment not covered by the claimed invention) shows a thermostabilizing effect beginning at 250 mM. Here optimum results are found for compositions when the concentration is in the range of between 1 to 2,5 M.
d) Maltose shows a thermostabilizing effect beginning at 500 mM. Optimum results are found for compositions when the concentration is in the range of between 1 M and 1,8 M,
   und
e) Lactulose (embodiment not covered by the claimed invention) shows a thermostabilizing effect beginning at 250 mM. Optimum results are found for compositions when the concentration is in the range of between 0,5 M and 1,7 M.

Corresponding results are shown and commented in the following chapter "Examples".

Furthermore, formulations with the enzymes lactate dehydrogenase and lysozyme as model proteins are also investigated here. The above-mentioned excipients are added in suitable concentrations and tested for their possible thermostabilizing effect. Correspondingly carried out experiments for stabilizing lactate dehydrogenase showed, that 4-hydroxy-proline (embodiment not covered by the claimed invention), L-ornithine (embodiment not covered by the claimed invention), lactobionic acid (embodiment not covered by the claimed invention) and potassium dihydrogenphosphate (embodiment not covered by the claimed invention) are good thermo-stabilizers, this means said excipients elevate the heat transition mid-point of LDH by 5°C or more.

In addition to this, in an embodiment not covered by the claimed invention, further potential thermo-stabilizers from the group of ionic liquids, four biodegradable ionic liquids are selected and prepared based on compounds that can be regularly found in the human body: Choline Chloride is found in bile, while Glucose, Sucrose, Sorbitol, and Glycerol are natural metabolites in the respective sugar or fat pathways. The corresponding ionic liquids are prepared as hydrated deep eutectitc mixtures in three steps. At first, 0.5 moles of Choline chloride are mixed with the respective amount of components of two sugars, sorbitol and glucose, and are used in an amount of 0.2 moles (ratio 5 : 2). Glycerol is used in an amount of 0.5 moles (ratio 1 : 1). Sucrose is used in an amount of 0.125 moles (ratio 4: 1) and 20% w/w H₂O is added.

Here the tests have shown, that Choline lactate and hydrated deep eutectic mixtures of Choline chloride with Sorbitol, Glucose, and Sucrose are good thermo-stabilizers for lactate dehydrogenase.

4- Hydroxyproline (embodiment not covered by the claimed invention), L-Ornitine (embodiment not covered by the claimed invention), Potassium dihydrogenphosphate (embodiment not covered by the claimed invention), Lactobionic acid (embodiment not covered by the claimed invention), and Meglumine (embodiment not covered by the claimed invention)show the required thermo-stabilizing effect on lactate dehydrogenase (LDH). Excipients that are identified as being suitable for thermostabilizing LDH are also used in experiments for thermostabilizing vaccine proteins. These screening experiments have shown that lactulose and maltose are good thermo-stabilizers in formulations with vaccine proteins.

In experiments not covered by the claimed invention with the enzyme lysozyme Choline lactate and hydrated deep eutectic mixtures of Choline chloride with Sorbitol, Glucose, and Sucrose respectively were identified as good thermostabilizers.

However, since the thermostabilizing effect of the added excipients was not satisfactory in every case, in addition tests were made if a synergistic effect of two excipients used in combination will improve the thermo-stabilizing effect of the two molecules. In the following a salt and an ionic liquid are combined with each other as well as with an osmolyte, and a sugar acid. All experiments are carried out using lactate dehydrogenase in the corresponding native formulation (50 mM Na Phosphate, 1 mM DTT (1,4-Dithiothreitol) at pH 7.6). The protein concentration is adjusted to a concentration of 0.05 mg/ml.

in this embodiment not covered by the claimed invention, it is found that in combination potassium dihydrogen phosphate and Lactobionic acid elicit a similar level of thermo-stability to LDH as can be expected from the individual components. The results suggest that if a corresponding salt and a sugar acid are compatible with each other in such protein solutions their use will result in an additive effect. This result is confirmed by use of a combination of potassium dihydrogen phosphate and 4-hydroxyl-proline which elicits a similar level of thermostabilizing effect on LDH.

In an embodiment not covered by the claimed invention, the results using a combination of potassium dihydrogen phosphate with a hydrated deep eutectic mixture of Choline Chloride and Sorbitol (molar ratio
5 : 2) are not so clear and can be summarized as follows:
The thermostabilizing effects of a salt and an ionic liquid behave
- compatible and additive when the salt is present in excess in comparison to the ionic liquid
- incompatible, but still thermo-stabilizing, when the ionic liquid is present in excess in comparison to the salt.

But in an embodiment not covered by the claimed invention, the effect of different combinations of a hydrated deep eutectic mixture of Choline Chloride and Sorbitol (molar ratio 5: 2) and Lactobionic acid in a composition of the model protein lactate dehydrogenase is different. In this context it may be concluded for the interaction of an ionic liquid and a sugar acid in this model composition that:
- The interaction of the ionic liquid and the sugar acid is concentration dependent.
- The interaction of the ionic liquid and the sugar acid is also dependent on the ratio ionic liquid to sugar acid.
- When a sufficiently large excess of ionic liquid is used, a synergistic effect of both excipients on the thermostability of a protein is observed.
- The ratio ionic liquid : sugar acid that is required for such a synergistic effect increases with an increasing fraction of the ionic liquid.
- The concentration of the sugar acid must be minimum 0.25 M in order to observe a synergistic effect of the two excipients.

In an embodiment not covered by the claimed invention, the effect of different combinations of a hydrated deep eutectic mixture of Choline Chloride and Sorbitol (molar ratio 5: 2) and 4-hydroxyl-proline, an amino acid, in the model protein composition comprising lactate dehydrogenase has a synergistic thermostabilizing effect on LDH over the entire ranges of concentrations and of ratios tested, causing 5% to 36% higher thermal stability than the effect which was expected for the two single components.

The effect of these combinations on vaccine proteins is similar.

In this third group of selected excipients it is found that according to the definition potassium dihydrogenphosphate, choline dihydrogenphosphate, choline lactate, and hydrated deep eutectic mixtures of Choline chloride with - Sorbitol, -Glucose, and -Sucrose respectively under some conditions can be classified as good thermostabilizer.

After evaluation of all tests carried out it is found that
1. there is a number of excipients which can stabilize protein formulations against the harmful effects of elevated temperatures.
   a. Stabilizing compounds/molecules or excipient belong to the group selected from 4-OH-Proline; L-Ornithine; Lactobionic acid; Potassium dihydrogenphosphate; 2-Hydroxyethyl-trimethylammonium L-(+)-lactate; hydrated deep eutectic mixtures of Choline chloride and a second component either: -Sorbitol or -Glucose or -Sucrose or -Glycerol; Lactose; Lactulose; Melibiose; Meglumine; Choline dihydrogenphosphate; TMAO; Betaine; Hydroxylectoine; Ectoine; Myo-Inositol; Citrullin; Ammonium acetate; Magnesium Gluconate; Mannitol; Tris(2-hydroxyethyl)methylammonium methylsulfate (embodiments not covered by the claimed invention) and Maltose.
   b. Especially efficient thermostabilizers are: 4-OH-Proline; L-Ornithine; Lactobionic acid; Potassium dihydrogenphosphate; 2-Hydroxyethyl-trimethylammonium L-(+)-lactate; hydrated deep eutectic mixtures of Choline chloride and a second component either: -Sorbitol or -Glucose or -Sucrose; Lactose, Lactulose, Melibiose, Meglumine, Choline dihydrogenphosphate, TMAO, Betaine, Hydroxylectoine (embodiments not covered by the claimed invention) and Maltose.
   c. The most efficient and broadly applicable excipients are TMAO, Choline dihydrogenphosphate, Lactulose, Melibiose (embodiments not covered by the claimed invention) and Maltose.
2. the required concentration range differs for each excipient and also the range in which each individual excipient
   a) stabilizes a specific protein
      and
   b) stabilizes a specific protein above the temperature by more than 5 ° C, at which the protein is still effective.
3. The experiments disclose that each of the excipients listed in Table 4 is able to stabilize proteins at temperatures higher than 60°C.
4. Said excipients are compatible with a broad spectrum of buffers used in biology/biochemistry (HEPES, Phosphate buffer, Histidine buffer, Citrate buffer)
5. Said excipients can be used in a pH range of between 6 to 8.
6. Said excipients are compatible with reducing agents (i. e. DTT).
7. Said excipients are compatible with chloride salt.
8. The experiments show that Melibiose (embodiment not covered by the claimed invention) and Maltose are suitable to suppress protein aggregation.
9. The tested excipients provide thermo-stabilization by stabilizing the tertiary structure of a protein, by stabilizing a molten globule state of a protein or by suppressing aggregation of dissolved monomers.
   [Molten globules are compact, partially folded conformations of proteins that have near-native compactness, substantial secondary structure, little detectable tertiary structure and increased solvent-exposed hydrophobic surface area relative to the native state. (Anthony L Fink, University of California, Santa Cruz, California, USA, published online 2001)]
10. The use of a combination of two excipients (embodiments not covered by the claimed invention) can have very different effects:
   a. A salt combined with a sugar acid shows an additive behavior: The resulting change in Tₘ is similar to the sum of the change in unfolding temperature elicited by the individual components.
   b. A salt combined with an osmolyte shows an additive behavior: The resulting change in Tₘ is similar to the sum of the change in unfolding temperature elicited by the individual components.
   c. A salt combined with an osmolyte show an additive behavior: The resulting change in Tₘ is similar to the sum of the change in unfolding temperature elicited by the individual components.
   d. A salt combined with hydrated deep eutectic mixtures of Choline chloride and Sorbitol shows a complex behavior:
      i.Shows an additive behavior while the salt is used in excess of the ionic liquid
      ii. Is still thermo-stabilizing but less efficient than sum of the two individual components, when the ionic liquid is used in excess of the salt
   e. A hydrated deep eutectic mixtures of Choline chloride and Sorbitol combined with Lactobionic acid:
      i.The interaction of the ionic liquid and the sugar acid is concentration dependent
      ii.The interaction of the ionic liquid and the sugar acid is also dependent on the ratio ionic liquid to sugar acid
      iii.When a sufficiently large excess of ionic liquid is used, we observe a synergistic effect of both excipients on the thermo-stability of a protein
      iv.The ratio ionic liquid to sugar acid that is required for such a synergistic effect increases with an increasing fraction of the ionic liquid
      v.The concentration of the sugar acid must be at least 0,25 M in order to observe a synergistic effect of the two excipients
   f. Hydrated deep eutectic mixtures of Choline chloride and Sorbitol in combination with an osmolyte/amino-acid have a synergistic thermo-stabilizing effect on LDH eliciting a 5%-36% greater thermo-stability than expected from the effect of the two individual components

The above described results, but also the aggregation suppression by the addition of melibiose (embodiment not covered by the claimed invention) and maltose are obtained by formulating a group of different proteins in test solutions to study the influence of the previously examined excipients. Selected proteins are from bacteria and animal origin. Among these proteins were enzymes, a pore former, and two inactivated toxins. One toxin was inactivated by a mutation, while the second was inactivated by formaldehyde cross-linking.

Since these results have been obtained with such different proteins, it can be inferred from these results that the addition of these selected excipients produce similar effects in formulations in which soluble proteins are contained as major component.

Another conclusion from these results is also admissible that these effects also occur when an additional component is covalently bound to the respective protein, for example if said protein is a protein-protein conjugate (e.g. Tetanus toxoid), a fusion-protein, a protein-polysaccharide conjugate, a protein-nucleic acid conjugate, a protein-DNA conjugate, a protein-RNA conjugate, a protein-dye conjugate, a protein-biotin conjugate or a protein-alum adsorbate.

The preparation of the specific protein formulations as disclosed herein can be carried out by methods as described above and as shown in the following examples.

The present description in combination with the given examples enables one of ordinary skill in the art to practice the present invention comprehensively.

The examples given below to illustrate the present invention will be commented upon in detail in connection with the results found in the following sections. These comments also serve to broadly describe the present invention in the context of the various protein formulations.

Even without further comments, it is therefore assumed that a person of ordinary skill in the art will be able to utilise the above description in the broadest scope.

If anything is unclear, it is understood that the publications and patent literature cited and known to the artisan should be consulted. Accordingly, cited documents are regarded as part of the disclosure content of the present description and are incorporated herein by reference.

For better understanding and in order to illustrate the invention, examples are presented below which are within the scope of protection of the present invention. These examples also serve to illustrate possible variants.

Furthermore, it goes without saying to one of ordinary skill in the art that, both in the examples given and also in the remainder of the description, the component amounts present in the compositions always only add up to 100% by weight or mol%, based on the composition as a whole, and cannot exceed this percentage, even if higher values could arise from the per cent ranges indicated. Unless indicated otherwise, % data are therefore % by weight or mol%, with the exception of ratios, which are shown in volume data.

### Examples

Here model proteins are used, such as lactate dehydrogenase (rabbit muscle) and lysozyme (chicken egg, white) as well as the vaccine proteins like Anthrax protective antigen (AnthraxPA), Tetanus toxoid and Diphtheria toxoid (CRM197) to demonstrate an effective thermostabilization by addition of one or more of the excipients, which are listed above. A liquid formulation is prepared comprising the proteins in a concentration in the range of between 0.05 to 1.8 mg/ml. Depicted below are the melting temperatures of these proteins in the presence of most efficient and most applicable thermostabilizers tested. The increase of the protein melting temperature is an indicator of increased thermal stability of the protein in the formulation.

### Materials and methods

**Table 1:**

| Excipient | grade | | Supplier | Article No. |
|---|---|---|---|---|
| 4-Hydroxyl-Proline | ≤99 % | | Sigma Aldrich | MKCB7394 |
| L-Ornithine | ≤99 % | | Sigma Aldrich | 5LBQ3983V |
| | | | | 5LBS8993 |
| Lactobionic acid | 97% | | Sigma Aldrich | BCBT8827 |
| | | | | BCBW9745 |
| Potassium dihydrogenphosphate | for analysis EMSURE^{®} ISO | | EMD Millipore | AM0655673528 |
| Lactulose | ≥98.0% | | Sigma Aldrich | BCBN5613 |
| | | | | SCBS9791 |
| Maltose | BioXtra, ≥99% | | Sigma Aldrich | SLBT8849 |
| | | | | SLBS9328 |
| Meglumine | low in endotoxins EMPROVE^{®} API Ph Eur, ChP, JP, USP | | EMD Millipore | 6143J608 |
| 2-Hydroxyethyl-trimethylammonium L-(+)-lactate aka choline lactate | 95% | | Sigma Aldrich | BCBR7035V |
| Sorbitol | Calbiochem | | EMD Millipore | 2838776 |
| Glukose | anhydrous EM PROVE^{®} EXPERT Ph Eur, BP, USP, ACS | | EMD Millipore | K8140448647 |
| Sucrose | EMPROVE^{®} bio Ph.Eur., BP, NF, JP / EMPROVE^{®} Essential Ph Eur, BP, JP, NF | | EMD Millipore | N017048754813 |
| | | | | K47855853645 |
| Ectoine | ≥ 94 % | | EMD Millipore | K47992200625 |
| | | | Ronacare | K4722500607 |
| Myo- Inositol | EMPROVE^{®} Essential Ph Eur, FCC, NF | | EMD Millipore | K44956531606 |
| Citrullin | ≥98% | | Sigma Aldrich | BCBT7042 |
| Ammonium acetate | for analysis EMSURE^{®} ACS, Reag. Ph Eur | | EMD Millipore | A0781316506 |
| | | | | BCBQ0228V |
| Magnesium Gluconate | ≥98% | | Sigma Aldrich | SLBR7216V |
| Mannitol | low in endotoxins suitable for use in solution EMPROVE^{®} API Ph Eur, BP, USP, JP | | EMD Millipore | FN1343403706 |
| | | | | M759903247 |
| Tris (2-hydroxyethyl) methylammonium methylsulfate (Me OSO₃) | ≥95% | | Sigma Aldrich | STBG2808V |
| Polysucrose 400 | powder | | Sigma Aldrich | WXBB5225V |
| Glycerol | anhydrous suitable for use as excipient EMPROVE^{®} exp Ph Eur, BP, JP, USP, E 422,FCC | | EMD Millipore | K81103536290 |
| Lactose | ≥99% total lactose basis for biochemistry | | Sigma Aldrich EMD Millipore | 1002428781 |
| | | | | FN1341860703 |
| Melibiose | ≥98% | | Sigma Aldrich | BCBS3427V |
| | | | | BCBR7124V |
| Choline | - | | io-li-tec | N00271.1.2-IL-0042 |
| dihydrogenphosphate | | | | Q00252.7-IL-0042 |
| Betaine | BioUltra, ≥99.0% | | Sigma Aldrich | BCBT9827 |
| Hydroxylectoine | ≥95% | | Sigma Aldrich | BCBT4444 |
| | | | | BCBX0515 |
| | | | | BCBV4503 |
| TMAO (Trimethylamine N-oxide dihydrate) | purum, ≥99.0% | | Sigma Aldrich | BCBS8627V |

**1) Osmolytes:** (embodiment not covered by the claimed invention) Example TMAO
a) A buffer is prepared consisting of a buffering agent (Sodium dihydrogenphosphate, HEPES, Histidine) and a pH of 7 or 7.5 or 8 is adjusted.
b) TMAO is dissolved in the buffer solution at a concentration of 4.2 M, the pH is readjusted to pH 7 or 7.5 or 8.
c) A Vaccine Protein, Anthrax Protective Antigen or CRM197 or Tetanus toxoid is added to the excipient. The protein concentration is 0.1 mg/ml. The excipient concentration is 4 M.
d) The melting temperature of the protein-excipient mixture is measured by differential scanning fluorimetry and compared with an excipient free protein solution
e) Result: TMAO increases the melting temperature of
   Anthrax Protective Antigen by 20°C
   Tetanus toxoid by 3°C
   Diphtheria toxoid by 7°C

**2) Ionic liquids:** (embodiment not covered by the claimed invention) Example Choline dihydrogenphosphate
a) Choline dihydrogenphosphate is dissolved in the water at a concentration of 4 M, the pH is adjusted to pH 7 or 7.5 or 8
b) A Vaccine Protein, Anthrax Protective Antigen or CRM197 or Tetanus toxoid is added to the excipient. The protein concentration is 0.1 mg/ml. The excipient concentration is 3 M
c) The melting temperature of the protein-excipient mix is measured by differential scanning fluorimetry and compared to an excipient free protein solution
d) Result: Choline dihydrogenphosphate increases the melting temperature of

| | |
|---|---|
| Anthrax Protective Antigen | by 19°C |
| Tetanus toxoid | by 5°C |
| Diphtheria toxoid | by 13°C |

**3) Salts:** (embodiment not covered by the claimed invention) Example Potassium dihydrogenphosphate
a) A buffer solution is prepared comprising a buffering agent (Sodium dihydrogen phosphate, HEPES, Histidine) and the pH is adjusted to a value of 7, 7.5 or 8.
b) Potassium dihydrogen phosphate is dissolved in the buffer solution at a concentration of 1.2 M, the pH is readjusted to pH 7 or 7.5 or 8.
c) A Vaccine Protein, Anthrax Protective Antigen or CRM197 or Tetanus toxoid is added to the excipient. The protein concentration is 0.1 mg/ml. The excipient concentration is 1 M.
d) The melting temperature of the protein-excipient mix is measured by differential scanning fluorimetry and compared to an excipient free protein solution.
e) Result: Potassium dihydrogenphosphate increases the melting temperature of

| | |
|---|---|
| Anthrax Protective Antigen | by 10°C |
| Tetanus toxoid | by 2°C |
| Diphtheria toxoid | by 5°C |

**4) Sugars:** (embodiment not covered by the claimed invention) Example Lactulose
a) A buffer is prepared consisting of a buffering agent (Sodium dihydrogenphosphate, HEPES, Histidine) and a pH of 7 or 7.5 or 8 is adjusted
b) Lactulose is dissolved in the buffer solution at a concentration of 2 M, the pH is readjusted to pH 7 or 7.5 or 8
c) A Vaccine Protein, Anthrax Protective Antigen or CRM197 or Tetanus toxoid is added to the excipient. The protein concentration is 0.1 mg/ml. The excipient concentration is 1.7 M
d) The melting temperature of the protein-excipient mix is measured by differential scanning fluorimetry and compared to an excipient free protein solution
e) Result: Lactulose increases the melting temperature of

| | |
|---|---|
| Anthrax Protective Antigen | by 18°C |
| Tetanus toxoid | by more than 20°C (apparatus does not allow for a precise measurement of Tm at such high temperatures) |
| Diphtheria toxoid | by 24°C |

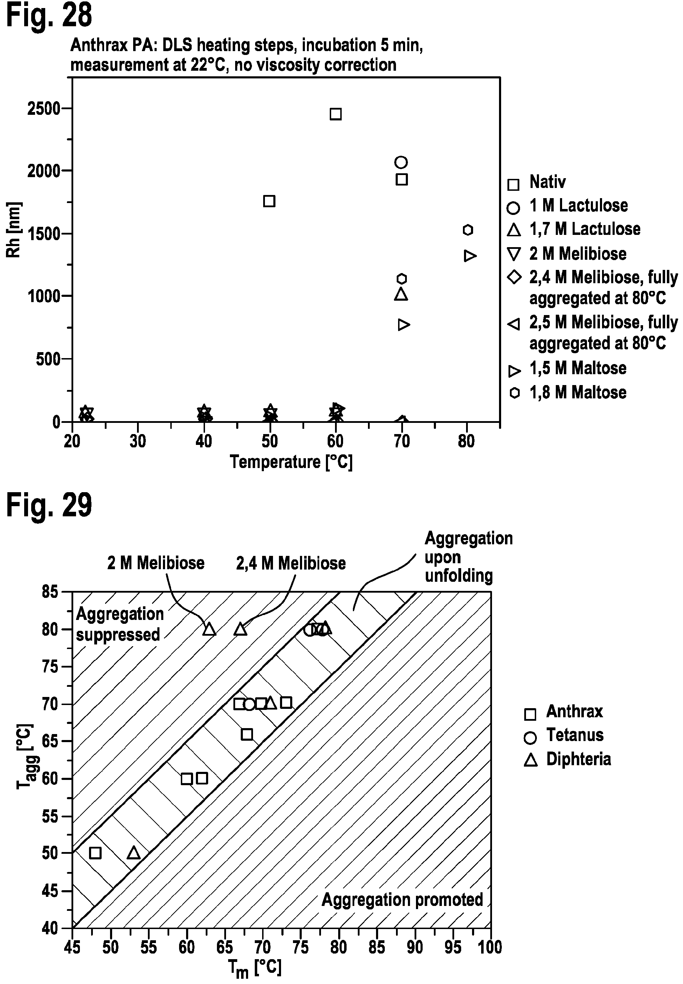

### Additional results: Aggregation prevention by Melibiose (Figures 28 and 29)

Melibiose is dissolved in the buffer solution at a concentration of 2.75 M and a pH of 7 or 7.5 or 8 is adjusted.

A vaccine protein suspension of Anthrax Protective Antigen or CRM197 or Tetanus toxoid is added to the excipient solution. The protein concentration is 0.1 mg/ml. The excipient concentration is 2 M or 2.4 M or 2.5 M.

### Result:

The aggregation of Diphtheria toxoid is suppressed to temperatures up to 80°C when using 2 M or 2.4M excipient. No aggregation is observed when 2,5 M excipient are used.

The aggregation of Tetanus toxoid is suppressed when 2.4 M or 2.5 M excipient are used.

### 5) Sugar acids and their salts: (embodiment not covered by the claimed invention)

### Example Lactobionic acid

a) A buffer is prepared consisting of a buffering agent (HEPES, Histidine) and a pH of 7 or 7.5 is adjusted
b) Lactobionic acid is dissolved in the buffer solution at a concentration of 0.6 M, the pH is readjusted to pH 7 or 7.5
c) A Vaccine Protein, Anthrax Protective Antigen or Tetanus toxoid is added to the excipient. The protein concentration is 0.1 mg/ml. The excipient concentration is 0.5 M
d) The melting temperature of the protein-excipient mix is measured by differential scanning fluorimetry and compared to an excipient free protein solution
e) Result: Lactobionic acid increases the melting temperature of

| | |
|---|---|
| Anthrax Protective Antigen | by 7°C |
| Tetanus toxoid | by 4°C |

### 6) Amino sugars: (embodiment not covered by the claimed invention) Example Meglumin

a) A buffer solution is prepared consisting of a buffering agent (Sodium Phosphate or Sodium Citrate) and a pH of 6 or 7.6 is adjusted
b) Meglumin is dissolved in the buffer solution at a concentration of 3 M, the pH is readjusted to pH 6 or 7.6 [if the pH is 7.6 1 mM DTT (1,4-Dithiothreitol) is added]
c) The protein, Lactate dehydrogenase or Lysozyme is added to the excipient solution. The protein concentration is 0.1 mg/ml. The excipient concentration is 1.5 M.
d) The melting temperature of the protein-excipient mix is measured by differential scanning fluorimetry and compared to an excipient free protein solution.
e) Result: Meglumine increases the melting temperature of

| | |
|---|---|
| Lactate dehydrogenase | by 3°C |
| Lysozyme | by 6°C. |

### 7) Amino alcohols: (embodiment not covered by the claimed invention) Example Mannitol

a) A buffer solution is prepared consisting of a buffering agent (Sodium Phosphate) and a pH of 6 or 7.6 is adjusted.
b) Mannitol is dissolved in the buffer solution at a concentration of 0.5 M, the pH is readjusted to pH 7.6, 1mM DTT is added.
c) A protein, Lactate dehydrogenase is added to the excipient solution in an amount resulting in a protein concentration is 0.1 mg/ml. The excipient concentration is 0.45 M.
d) The melting temperature of the protein-excipient mix is measured by differential scanning fluorimetry and compared to an excipient free protein solution.
e) Result: Mannitol increases the melting temperature of

| | |
|---|---|
| Lactate dehydrogenase | by 2°C. |

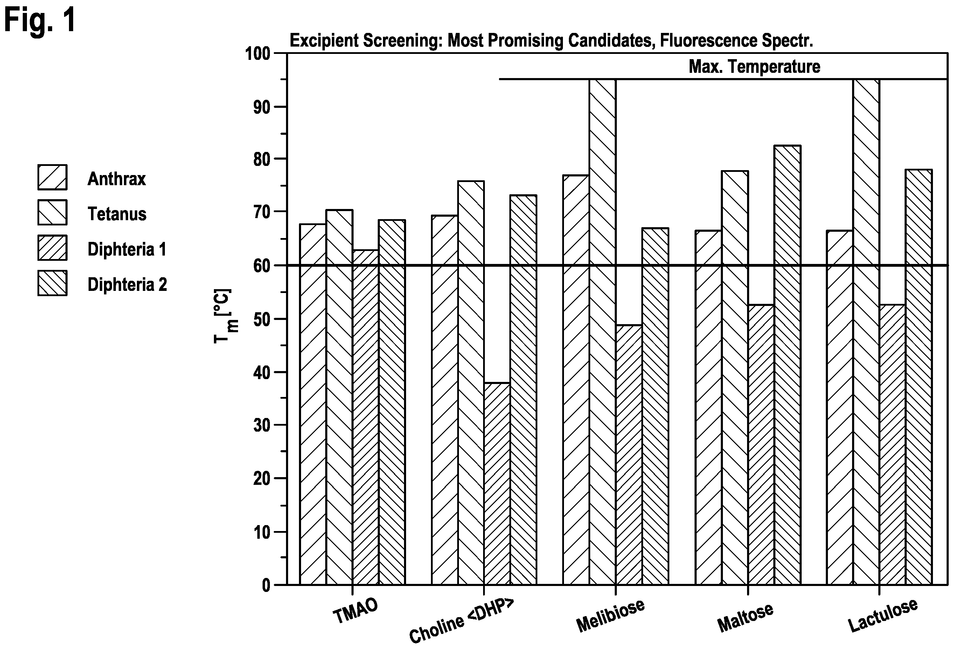

Figure 1 gives a summary of results of added excipients showing the best performance. This means, that results are summarized of added excipients which inflicted the largest change in melting temperature of tested proteins, and which show the broadest applicability. These excipients are suitable as stabilizer for all three vaccine proteins tested. Surprisingly, it has been found that these are the following excipients, which in themselves belong to very different substance groups:
f) TMAO (embodiment not covered by the claimed invention) shows a thermostabilizing effect beginning at a concentration of 250 mM. Optimum stabilization results are obtained at a concentration in the range of 1 to 4 M in solution.
g) Choline dihydrogenphosphate (embodiment not covered by the claimed invention) shows a thermostabilizing effect starting with a 1 molar solution. Optimum results are found for compositions when the concentration is in the range of 2 to 4 M.
h) Melibiose (embodiment not covered by the claimed invention) shows a thermostabilizing effect beginning at 250 mM. Here optimum results are found for compositions when the concentration is in the range of between 1 to 2,5 M.
i) Maltose shows a thermostabilizing effect beginning at 500 mM. Optimum results are found for compositions when the concentration is in the range of between 1 M and 1,8 M, und
j) Lactulose (embodiment not covered by the claimed invention) shows a thermostabilizing effect beginning at 250 mM. Optimum results are found for compositions when the concentration is in the range of between 0,5 M and 1,7 M.

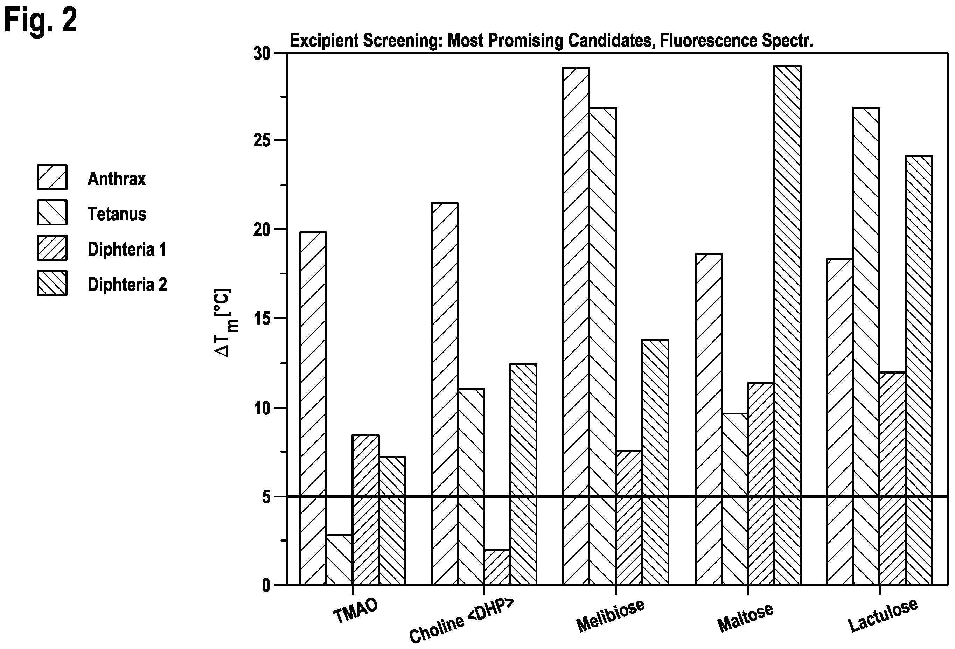

Figure 2 gives a summary of results about the change in melting temperature inflicted by the respective excipient under optimal conditions:

### Experimental setting and considerations

In order to evaluate which additives or excipients are best suited for thermo-stabilization and in what amounts they have to be added to produce the desired effect, test formulations with AnthraxPA as a vaccine were prepared and examined over three days. The detailed formulation compositions are summarized in a Table 2 at the end of this chapter. The aim of these experiments is to find the most suitable excipient or mixture of excipients for thermo-stabilization of protein compositions over several days.

Since to date stabilizing agents have to be used at high concentrations, such compositions cannot be used to prepare pre-filled syringes because patients would likely not tolerate the stabilizing agent. A dilution step is therefore required prior to administration to the patients. Another objective of these experiments is also to determine the highest likely tolerable levels of stabilizing agents for use in vitro assays. Based on the results of the experiments, the protein concentration can be adjusted and a vaccine stock solution can be generated, which can provide both heat protection for the solute protein but can still be effectively administered to the patient after dilution.

For example, it is found that Melibiose (embodiment not covered by the claimed invention) can very likely be tolerated by a patient up to a concentration of 250 mM. From the previous screenings it is known, that at least 2.25 M Melibiose is required to stabilize AnthraxPA at high temperatures. Therefore, the protein concentration is increased by a factor of 2250 mM (stock) / 250 mM (final) = 9. Therefore, at least an amount of a nine-fold vaccine stock formulation is required.

According to these considerations, test formulations are prepared for the study of TMAO (embodiment not covered by the claimed invention), choline dihydrogen phosphate (embodiment not covered by the claimed invention), lactulose (embodiment not covered by the claimed invention), maltose and melibiose (embodiment not covered by the claimed invention) as temperature stabilizing additives. Fluorescence spectroscopy is used to verify whether all protein samples are natively folded and that the selected excipients can also thermo-stabilize elevated protein concentrations (up to 1.6 mg/ml). Then incubation is carried out at the following temperatures:

| | |
|---|---|
| 4°C: | Native (no excipient): These are control samples that do not change. |
| 50°C: | Native (no excipient), TMAO, Choline dihydrogenphosphate, Lactulose, Maltose |
| 55°C: | TMAO, Choline dihydrogenphosphate, Lactulose, Melibiose, Maltose |
| 60°C: | TMAO, Choline dihydrogenphosphate, Lactulose, Melibiose, Maltose |
| 65°C: | Melibiose |

Melibiose (embodiment not covered by the claimed invention) is tested at higher temperatures than all other excipients because it elicits the higher thermostability based on the transition mid-point.

After each incubation period of 6 hours, 24 hours, 32 hours, 48 hours and 72 hours, a small sample is taken and the structural state and stability of the comprising protein is evaluated by means of fluorescence spectroscopy.

**Table 2: Summary of the test formulations used herein.**

| **Anthrax PA** | Native formulation: 50 mM/NaCl, 5mM HEPES, pH 7,5 (Do not vortex!) | |
|---|---|---|
| Protein stock 11 mg/ml | | |
| Port. Excipient | Excipient concentration [M] | Protein concentration [mg/ml] |
| None | - | 1 |
| Trimethylamine N-oxide (TMAO) | 3,1 | 1,6 |
| Choline dihydrogenphosphate | 2,3 | 1,2 |
| Lactulose | 1,8 | 1 |
| Melibiose | 2,25 | 1 |
| Maltose | 2 | 1 |

### Analytical parameters

The results presented in the following sections are based on two parameters determined by fluorescence spectroscopy. The change of these two parameters is monitored over time to determine if AnthraxPA is still native or whether it is damaged. Both readouts as described below are required to find out whether the comprising protein remains stable throughout the duration of the experiment.

### AnthraxPA stabilized at temperatures above 50°C

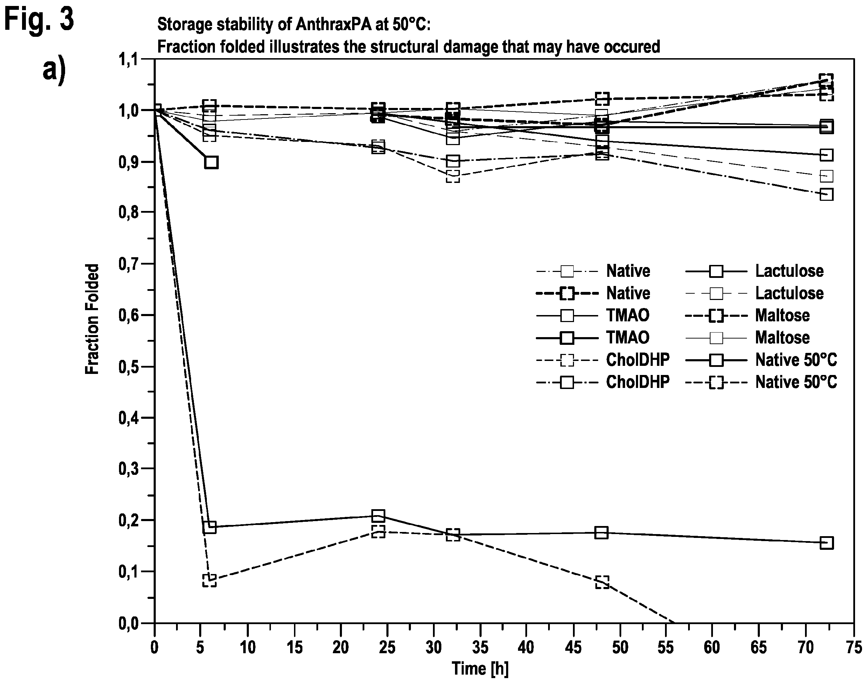

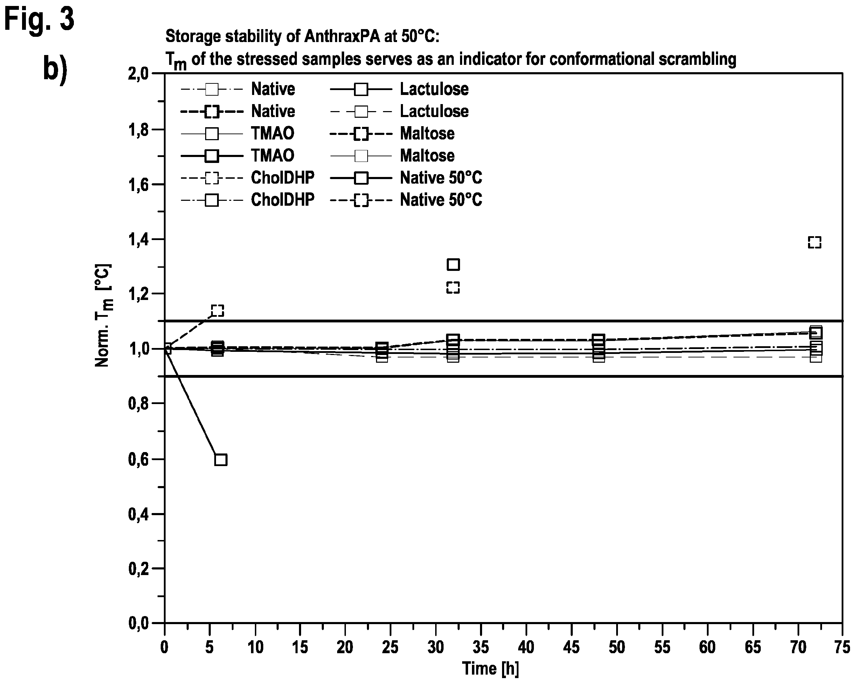

In its native formulation AnthraxPA has an unfolding transition temperature of 48°C, which would not permit storage of the protein at 50°C or above. Figure 3 shows the storage stability of AnthraxPA at 50°C assayed by fluorescence spectroscopy.

Figure 3: Stability of AnthraxPA at 50°C.

Figure 3a) (Left) Fraction folded. Natively formulated AnthraxPA shows an immediate decline in natively folded protein. All thermo-stabilizers tested prevent such a decay.

Figure 3b) (Right) Normalized transition temperature. Data between the red lines indicates that the protein is stable.

AnthraxPA without thermo-stabilizing excipients denatures immediately. All selected excipients stabilize and allow storage for 72 hours at 50°C.

Figure 4 shows the storage stability of AnthraxPA at 55°C.

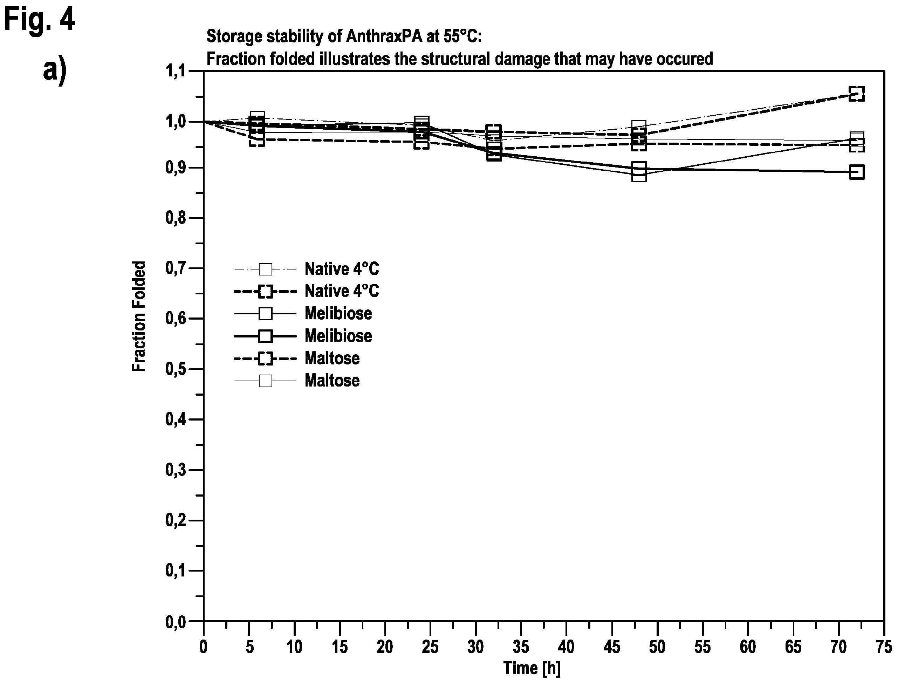

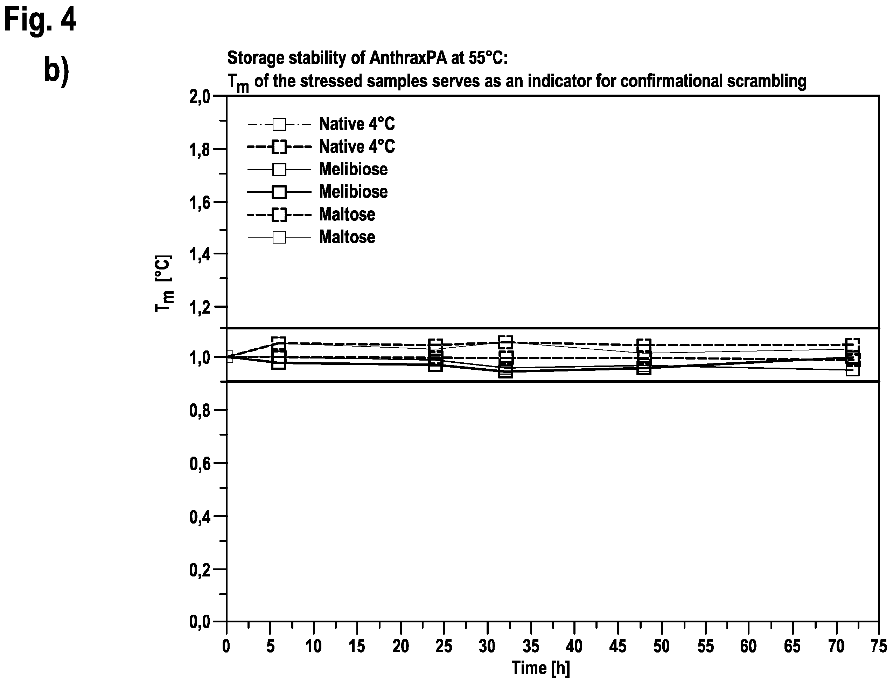

Figure 4: Stability of AnthraxPA at 55°C.

Figure 4a) (Left) Fraction folded.

Figure 4b) (Right) Normalized transition temperature. Data between the red lines indicate that the protein is stable.

When the test formulations are incubated at 55°C, it is found that Melibiose and Maltose are both capable of eliciting sufficient thermo-stabilization to allow for storage at 55°C over a period of three days.

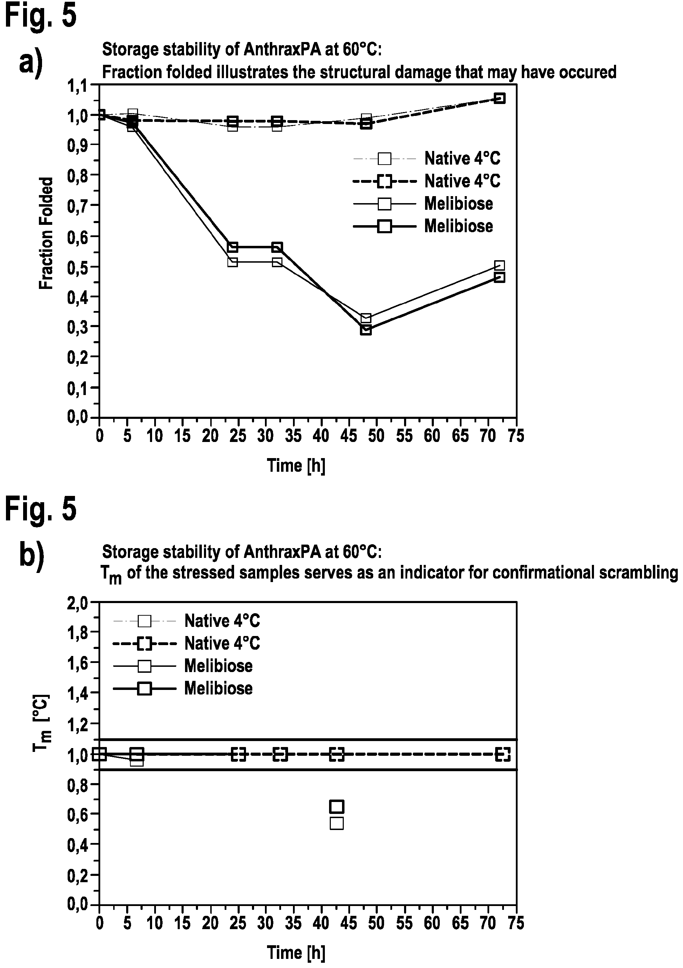

In addition, AnthraxPA was incubated at 60°C for three days. The referring results are shown in Figure 5.

Figure 5: Stability of AnthraxPA at 60°C.

Figure 5a) (Left) Fraction folded. Data below the red line indicates a stable protein.

Figure 5b) (Right) Normalized transition temperature. Data between the red lies indicates that the protein is stable.

At 60°C the thermostabilizing ability of the excipients used herein comes to an end. Only Melibiose is able to allow for a brief exposure to 60°C for over 6, but less than 24 hours.

### CRM197 stabilized at temperatures above 50°C

In its native formulation CRM197 shows a biphasic unfolding transition when assayed with fluorescence spectroscopy. The initial transition relates to a localized loss of structure while on a global scale the protein remains structurally intact. While the excipients used herein have an effect on the global structural stability of CRM, the more local loss of structure cannot be prevented. Therefore, the same criteria cannot be used as for AnthraxPA when studying the stability properties of CRM.

For data interpretation the following two modifications are required: First, the median unfolding temperature cannot be used as a readout. It is already established that conformational scrambling occurs due to the local loss of structure that cannot be stabilized by the excipients used herein. Furthermore, changes in this initial transition may affect the determination of the second unfolding temperature and lead to an erroneous assessment of the global structural state based thereon. Secondly, the extent of damage inflicted on the thermoliable region of CRM is affecting the fluorescence emission ratio 350 nm to 330 nm. Therefore, the extend of structural damage is likely to be an upper limit rather than the actual degree of loss of structure. In order to evaluate the effect of the excipients tested herein, all results are compared to the protein in its native formulation incubated and denatured at 50°C.

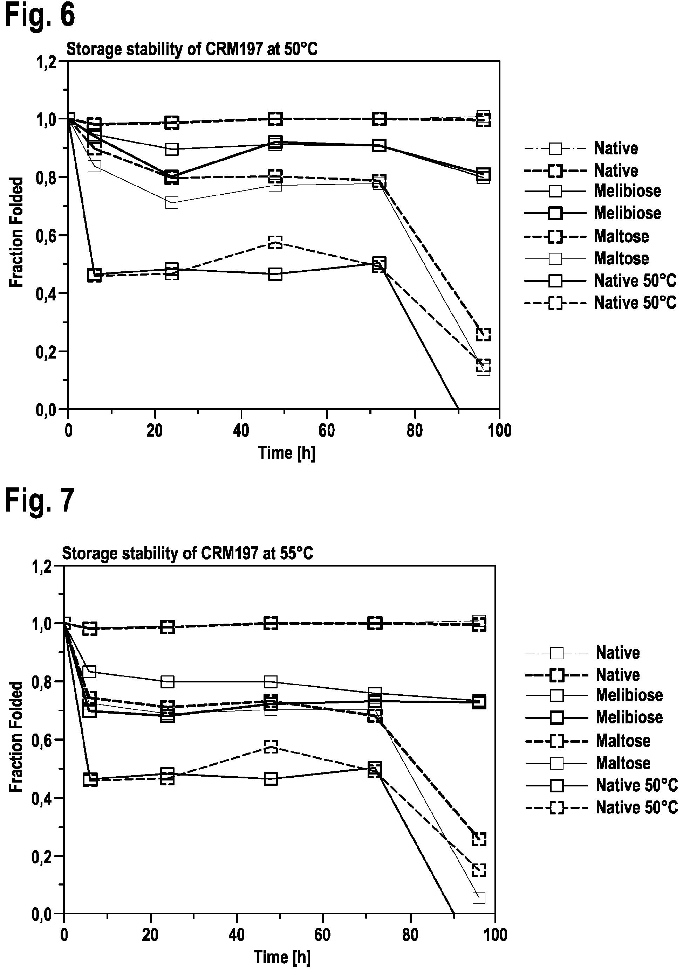

In its native formulation CRM197 has a global unfolding temperature of 53°C, which does not permit extended storage at 50°C. Therefore, the negative control will be natively formulated CRM incubated at 50°C. In Figure 6 the effect of Melibiose and Maltose are shown on the thermal stability of CRM.

Figure 6: Stability of CRM197 at 50°C. Melibiose (embodiment not covered by the claimed invention) and Maltose clearly suppress thermally induced unfolding for 3-4 days.

The results show that Maltose stabilizes CRM197 for 72 hours at 50°C. Melibiose stabilizes CRM for 96 hours at 50°C.

Figure 7 shows the storage stability of CRM197 at 55°C.
- Figure 7:: Stability of CRM197 at 55°C. Melibiose (embodiment not covered by the claimed invention) and Maltose clearly suppress thermally induced unfolding for 3-4 days.

The results show that Maltose stabilizes CRM197 for 72 hours at 55°C. Melibiose stabilizes CRM for 96 hours at 55°C.

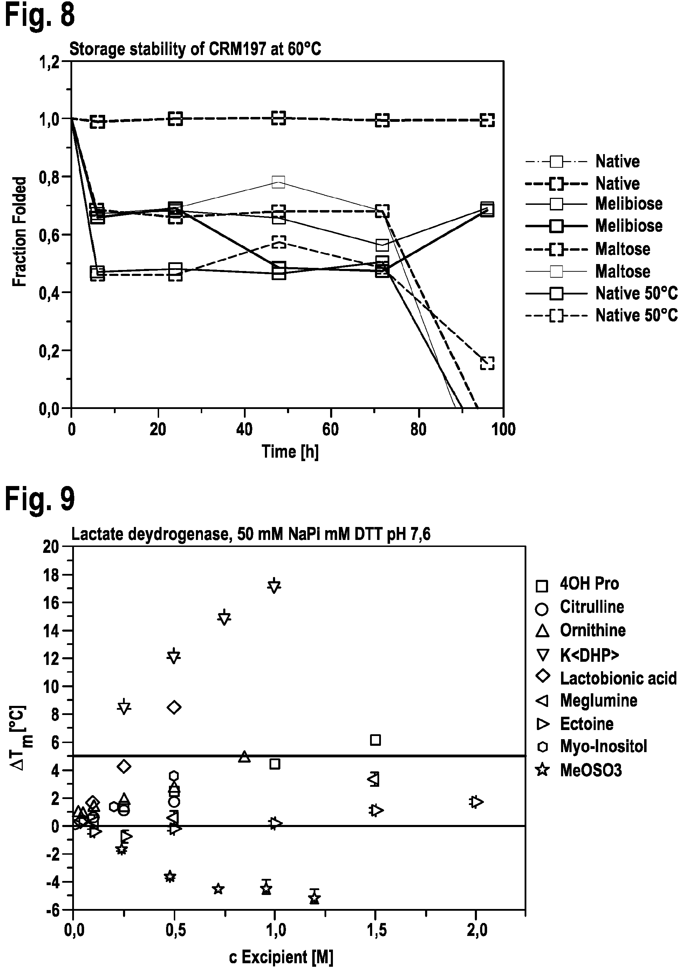

Figure 8 in turn shows the storage stability of CRM197 at 60°C.
- Figure 8:: Stability of CRM197 at 60°C. Melibiose (embodiment not covered by the claimed invention) and Maltose clearly suppress thermally induced unfolding for 3-4 days.

While it is noticeable that the fraction folded for 6 hours is greater at 60°C compared to 55°C or 50°C, there is still a clear difference to the natively formulated and fully denatured CRM. The results of these tests show that Maltose stabilizes CRM197 for 48 hours at 60°C. Melibiose stabilizes CRM for 24 hours at 60°C.

### Further experiments for thermostabilizing enzymes as protein

As described for the previous experiments and investigations with AnthraxPA and CRM197, formulations with the enzymes lactate dehydrogenase and lysozyme as model proteins are also investigated here. The above-mentioned excipients are added in suitable concentrations and tested for their possible thermostabilizing effect.

### a. Preparation of ionic liquids based on Choline Chloride (embodiment not covered by the claimed invention)

Further potential thermo-stabilizers from the group of ionic liquids, four biodegradable ionic liquids are selected and prepared based on compounds that can be regularly found in the human body: Choline Chloride is found in bile, while Glucose, Sucrose, Sorbitol, and Glycerol are natural metabolites in the respective sugar or fat pathways.

The ionic liquids are prepared as hydrated deep eutectic mixtures in three steps. At first, 0.5 moles of Choline chloride are mixed with the respective amount of components of two sugars, sorbitol and glucose, and are used in an amount of 0.2 moles (ratio 5: 2). Glycerol is used in an amount of 0.5 moles (ratio 1: 1). Sucrose is used in an amount of 0.125 moles (ratio 4: 1). Then the prepared mixture is heated to 100°C for two hours while stirring. While neither of the substances, except Glycerol and Sorbitol, would adopt a liquid form at this temperature, these particular mixtures begin to melt. Subsequently, 20% w/w is added to the mixture and when the solid substance is fully melted the generated ionic liquids are cooled down to room temperature where they maintain their liquid form.

In order to compare the amount of substance used for thermo-stabilization with ionic liquids prepared in this manner to other excipients that are merely dissolved in water/buffer the volume of the resulting liquid is determined and the concentration of Choline chloride therein is calculated as if it were dissolved. Typically, about 100-150 ml of ionic liquid are prepared representing a molar concentration of 3.3 - 4 M Choline chloride.

### b. Experimental approach

As described before the unfolding transition mid-point of comprising proteins is determined by fluorescence spectroscopy, this means by Nano-Differential Scanning Fluorimetry (nanoDSF) allowing for a high throughput screening.

Here the proteins are used in concentrations of 0.05-0.1 mg/ml, while excipients are titrated up to concentrations close to their solubility in the applied buffer solution. Then an appropriate amount of protein solution is mixed with an appropriate amount of excipient solution and the mixture is equilibrated for 15 minutes at room temperature.

The prepared protein solution is heated with a heating rate of 1 °C/minute and the ratio of the fluorescence emission at 350/330 nm is recorded and plotted vs. temperature. Typically, the resulting curve resembles a Boltzmann function and the inflection point of this curve represents the mean unfolding temperature Tₘ.

From this curve the change in Tₘ determined by subtracting T_{m native} from T_{m excipient} formulation, which yields to dTₘ.

By definition, an excipient eliciting a dTₘ of >5°C is a good stabilizer. If dTₘ is <5°C and >0°C the excipient is defined as a poor stabilizer. A negative dTₘ identifies a molecule as a protein destabilizer.

### c. Results using Lactate dehydrogenase as a model enzyme

Lactate dehydrogenase (LDH) is formulated into 50 mM Na Phosphate, 1 mM DTT at pH 7.6. In order to probe for the effect of a molecule on LDH, the said molecule is added at various concentrations to the protein formulated in exactly this buffer. The protein used in a concentration of 0.05 mg/ml.

Figure 9: shows the effect of excipient candidates on the mean unfolding temperature of LDH. The black line indicates dTₘ =0°C. The red line indicates dTₘ = 5°C. Molecules that are able to stabilize LDH by more than 5°C are subjected to a second screening with a different model protein.

Figure 9 shows a first set of excipients (embodiments not covered by the claimed invention), at this stage from a very diverse group of compounds, and their effect on the melting temperature of LDH.
4-hydroxy-proline, ornithine, lactobionic acid and Potassium dihydrogenphosphate are, according to the definition, good thermo-stabilizers, while others, such as ectoine, myo-inositol, and citrullin, are however, not suitable for the tested purposes due to the relatively small effect on the Tₘ of LDH.

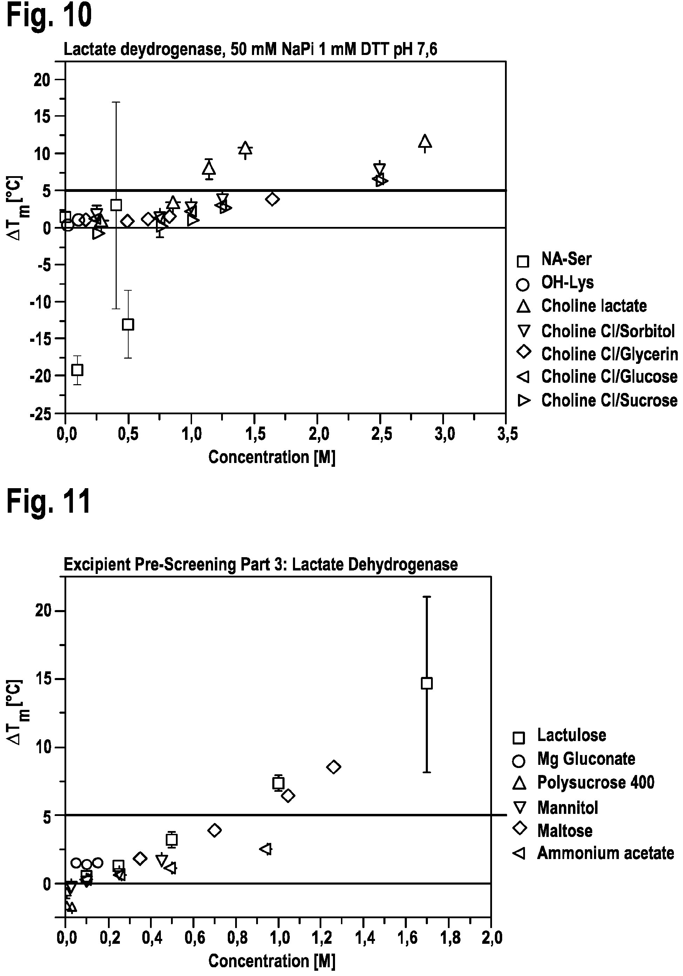

Figure 10 shows the change in the melting temperatures dTₘ of LDH, which are determined with the addition of Na-Ser, OH-Lys, choline lactate, choline Cl / sorbitol, choline Cl / glycerol, choline Cl / glucose, choline Cl / sucrose (embodiments not covered by the claimed invention).

Figure 10: Effect of excipient candidates on the mean unfolding temperature of LDH. The black line indicates dTₘ =0°C. The red line indicates dTₘ = 5°C. Molecules that are suitable to stabilize LDH by more than 5°C are subjected to a second screening with a different model protein. In order to use the same plot for the hydrated deep eutectic liquids as for the other excipients, the molar concentration of choline chloride was estimated based on the weight of material used and the volume of the prepared liquid.

In this set of excipients choline lactate and hydrated deep eutectic mixtures of choline dihydrogenphosphate with sorbitol, glucose, and sucrose respectively were identified as good thermo-stabilizers and advance to the second pre-screening step.

In the next tests the influence of lactulose, Mg gluconate (embodiment not covered by the claimed invention), Polysucrose 400 (embodiment not covered by the claimed invention), Mannitol (embodiment not covered by the claimed invention), Maltose and ammonium acetate (embodiment not covered by the claimed invention) on the thermostability of LDH is tested. These excipients that are identified as being suitable for thermostabilizing LDH herein. Figure 11: shows the effect of lactulose, Mg gluconate, Polysucrose 400, Mannitol, Maltose and ammonium acetate on the on the mean unfolding temperature of LDH. The black line indicates

dTₘ = 0°C. The red line indicates dTₘ = 5°C. Molecules that are able to stabilize LDH by more than 5°C are subjected to a second screening with a different model protein.

Especially Lactulose (embodiment not covered by the claimed invention) and Maltose are identified as good thermo-stabilizers. Ammonium acetate, Magnesium Gluconate, and Mannitol (embodiments not covered by the claimed invention) are also identified as protein-stabilizers.

### d. Results using Lysozyme as a model enzyme

Lysozyme is formulated 20 mM Na Citrate, 55 mM NaCl, 1 mM DTT at pH 6.0. In order to probe for the effect of a molecule on Lysozyme, said molecule is added at various concentrations to the protein formulated in exactly this buffer. A protein is used at a concentration of 0.1 mg/ml.

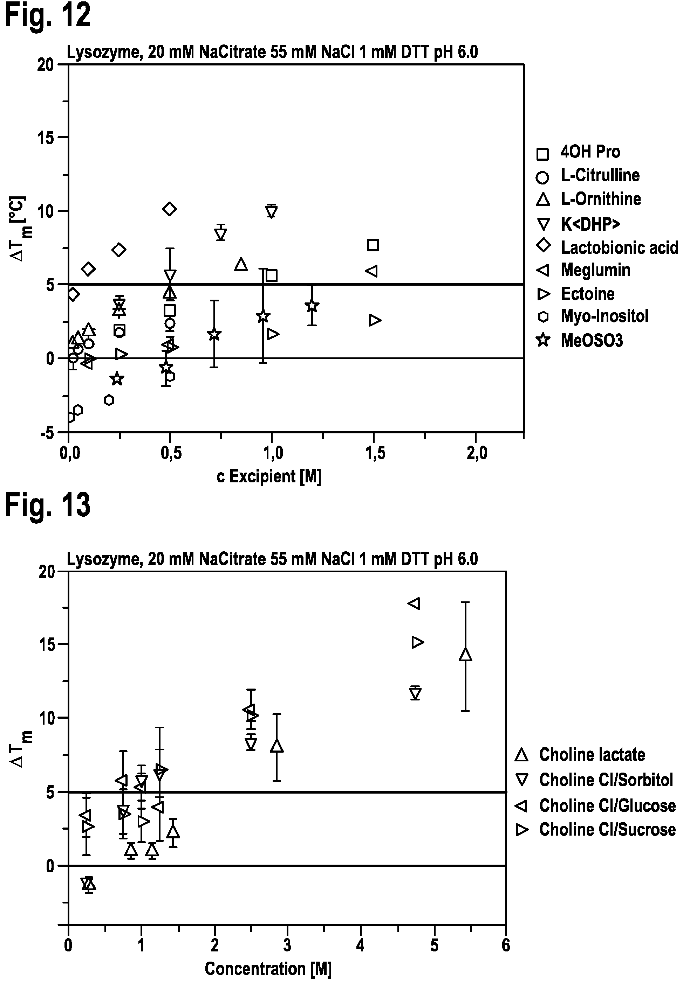

Figure 12: Effect of Hydroxy-Proline, L-Citrulline, L-Ornitine, potassium dihydrogen phosphate, lactobionic acid, meglumine, ectoine, myo-inositol and Methylsulfonat on the mean unfolding temperature of Lysozyme. The black line indicates dTₘ = 0°C. The red line indicates dTₘ = 5°C. (embodiment not covered by the claimed invention)

From this set of molecules Hydroxyproline, L-Ornitine, Potassium dihydrogenphosphate, Lactobionic acid, and Meglumin show the required thermo-stabilizing effect. Ectoine, Tris(2-hydroxyethyl)methylammonium methylsulfate (MeOSO3 ), and Citrullin do not stabilize Lysozyme sufficiently.

Figure 13 shows the results of the second group of substances tested in the with Lysozyme: ionic liquids.

Figure 13: Effect of excipient candidates on the mean unfolding temperature of LYZ. The black line indicates dTₘ =0°C. The red line indicates dTₘ = 5°C. Molecules that are able to stabilize LYZ by more than 5°C are identified as good thermostabilizers according the the previously stated definition. In order to use the same type of plot for the hydrated deep eutectic liquids as for the other excipients, the molar concentration of choline chloride is estimated based on the weight of material used and the volume of the prepared liquid.

Among the excipient candidates tested here Choline lactate and hydrated deep eutectic mixtures of Choline Dihydrogenphosphate with Sorbitol, Glucose, and Sucrose respectively were identified as good thermostabilizers (embodiment not covered by the claimed invention).

A summary of the concentration range used for each excipient that stabilized at least one of the model proteins by 5°C or more can be found in the Table 3. Typically, the molar excipient concentration is given. For ionic liquids, the volume percent of the excipient used are given, since the molar concentration of only one of the components would be misleading.

**Table 3:**

| pre-screening | | | | | | |
|---|---|---|---|---|---|---|
| Excipient | Concentration range used | | Concentration range thermos-stabilizer | | | |
| | [M] | | [M] | | | |
| | | | >0 °C | | min. 5 °C | |
| | LDH | Lyz | LDH | Lyz | LDH | Lyz |
| 4-Hydroxyl Proline | 0.25-1.5 | 0.25-1.5 | 0.25-1.5 | 0.25-1.5 | 0.25-1.5 | 0.25-1.5 |
| L-Ornithine | 0.025-0.85 | 0.025-0.85 | 0.025-0.85 | 0.025-0.85 | 0.85 | 0.85 |
| Lactobionic acid | 0.025-0.5 | 0.025-0.5 | 0.025-0.5 | 0.025-0.5 | 0.5 | 0.1-0.5 |
| Potassium dihydrogenphosphate | 0.25-1.0 | 0.25-1.0 | 0.25-1.0 | 0.25-1.0 | 0.5-1 | 0.5-1 |
| Lactulose | 0.1-1.7 | N.A. | 0.1-1.7 | N.A. | 1-1.7 | N. A. |
| Maltose | 0.5-1.8 | N.A. | 0.5-1.8 | N.A. | 1.5-1.8 | N.A. |
| Meglumine | 0.1-1.5 | 0.1-1.5 | 0.1-1.5 | 0.1-1.5 | none | 1.5 |
| 2-Hydroxy-ethyl-trimethylammonium L-(+)-lactate/ | 5-50% | 5-50% | 5-50% | 15-95% | 20-50% | 50-95% |
| Choline chloride Choline chloride/Sorbitol molar ratio 5:2 | 5-50% | 5-50% | 5-50% | 15-95% | 50% | 20-95% |
| Choline chloride/ Glucose molar ratio 5:2 | 5-50% | 5-95% | 5-50% | 5-95% | 50% | 15-95% |
| Choline chloride/Sucrose molar ratio 4:1 | 5-50% | 5-50% | 5-50% | 5-95% | 50% | 25-95% |
| Ectoine | 0.1-2 | 0.1-1.5 | 1-2 | 0.25-1.5 | none | none |
| Myo-Inositol | 0.01-0.5 | 0.01-0.5 | 0.5-0.5 | Destabilizer | none | none |
| Citrullin | 0.025-0.5 | 0.025-0.5 | 0.05-0.5 | 0.05-0.5 | none | none |
| Ammonium acetate | 0.1-0.85 | DNQ | 0.1-0.85 | DNQ | none | DNQ |
| Magnesium Gluconate | 0.05-0.15 | DNQ | 0.05-0.16 | DNQ | none | DNQ |
| Mannitol | 0.025-0.45 | DNQ | 0.1-0.45 | DNQ | none | DNQ |
| Tris (2-hydroxy-ethyl)methylammonium methyl-sulfate (MeOSO3) | 0.24-1.2 | 0.24-1.2 | Destabilizer | 0.7-1.2 | none | none |
| Polysucrose 400 | 10-85 g/l | DNQ | 5-50% | DNQ | none | DNQ |
| Cholin chloride/Glycerol molar ratio 1:1 | 5-50% | DNQ | 5-50% | DNQ | none | DNQ |

| | | | | | | |
|---|---|---|---|---|---|---|
| DNQ: Did not qualify none: no tested concentration elicited desired thermos-stability | | | | | | |

### Results showing the efficiency of Excipient Combinations using Lactate dehydrogenase (embodiment not covered by the claimed invention)

For an initial feasibility-assessment the compatibility of various excipients in combination with each other are tested. The aim is to elucidate if a synergistic effect of two excipients used in combination will improve the thermo-stabilizing effect of the two molecules. A salt and an ionic liquid are combined with each other as well as with an osmolyte, and a sugar acid. All experiments are carried out using lactate dehydrogenase in the corresponding native formulation (50 mM Na Phosphate, 1 mM DTT at pH 7.6). The protein concentration is adjusted to a concentration of 0.05 mg/ml. LDH is added to a solution comprising two excipients in various ratios and concentrations. The prepared compositions are incubated at room temperature for 15 minutes. Then the mean unfolding temperature of LDH is measured with a heating rate of 1°C/min using nano differential scanning fluorimetry as described above.

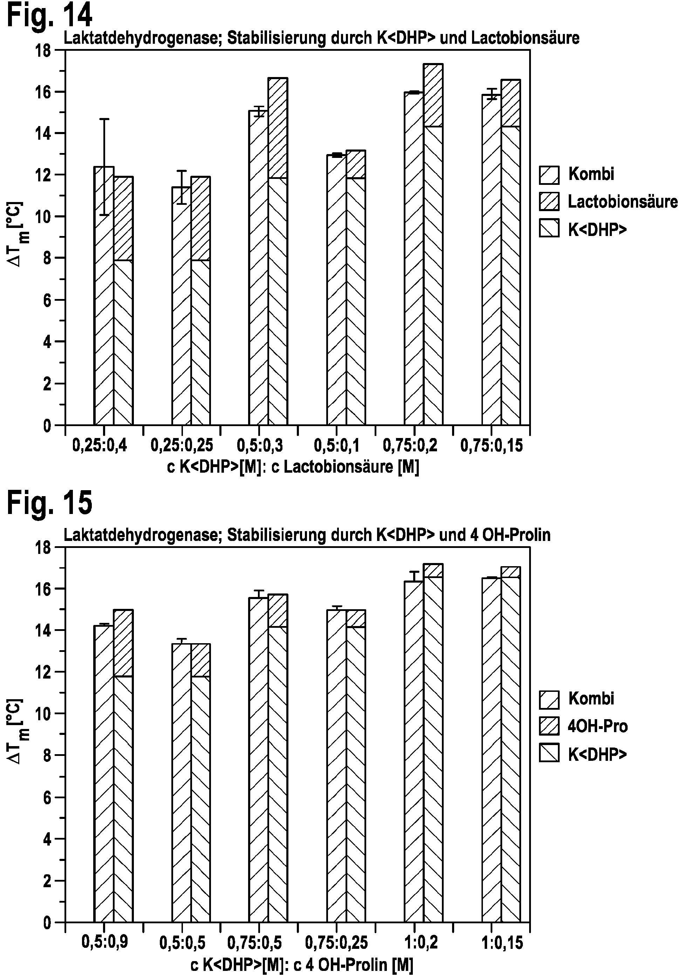

Figure 14 summarizes the results for the combination of potassium dihydrogen phosphate with Lactobionic acid

Figure 14: Effect of different combinations of potassium dihydrogen phosphate and Lactobionic acid on the model protein lactate dehydrogenase - in blue the measured change in Tₘ of the combined formulation. Stacked in black and red are the changes is Tₘ determined for the individual excipients separately. A synergistic effect would be detected if the blue bar surpasses the stacked bar of black and red.

It is found that in combination potassium dihydrogen phosphate and Lactobionic acid elicit a similar level of thermo-stability to LDH as can be expected from the individual components. In relative terms, when using this combination a thermo-stabilizing effectiveness can be observed between 90% and 104%.

This leads to the conclusion that a corresponding salt and a sugar acid are compatible in such protein solutions and lead to an additive effect.

Figure 15 summarizes the results for the combination of potassium dihydrogen phosphate and 4-OH-Proline (embodiment not covered by the claimed invention).

Figure 15: Effect of different combinations of potassium dihydrogen phosphate and Lactobionic acid in the model protein lactate dehydrogenase. In blue the measured change in Tₘ of the combined formulation. Stacked in black and red are the changes is Tₘ determined for the individual excipients separately. One would have to conclude to a synergistic effect, if the blue bar would surpass the stacked bar of black and red.

It is found that a combination potassium dihydrogen phosphate and 4-OH-Proline elicits a similar level of thermostabilizing effect on LDH as it could be expected if the individual components were added. In relative terms, when these compounds are used in combination a thermo-stabilizing effectiveness in the range between 95% to 100% of can be observed.

Therefore, it is concluded that the thermo-stabilizing effects of a salt and an osmolyte are compatible and act in an additive manner.

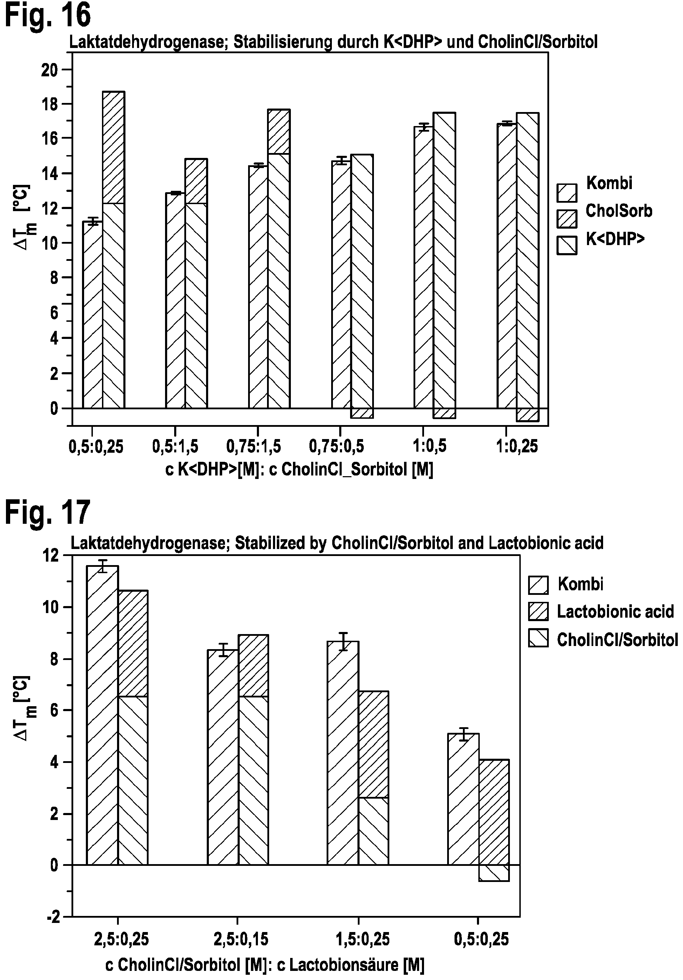

Figure 16 summarizes the results for the combination of potassium dihydrogen phosphate and Choline chloride/Sorbitol (embodiment not covered by the claimed invention)

Figure 16: Effect of different combinations of potassium dihydrogen phosphate and a hydrated deep eutectic mixture of Choline Chloride and Sorbitol (molar ratio 5: 2) in the model protein lactate dehydrogenase. In blue the measured change in Tₘ of the combined formulation. Stacked in black and red are the changes is Tₘ determined for the individual excipients separately. One would have to conclude to a synergistic effect, if the blue bar would surpass the stacked bar of black and red.

Here, it is observed, that a combination of potassium dihydrogen phosphate with a hydrated deep eutectic mixture of Choline Chloride and Sorbitol (molar ratio 5/2) are incompatible when the latter is used in excess in comparison to the former. With an excess of the ionic liquid in comparison to the salt, an effectiveness of 58% to 81% is found. However, when the salt is added in excess to the ionic liquid, the thermo-stabilizing effect of both is additive in the range of 99-102 %.

Accordingly, the thermostabilizing effects of a salt and an ionic liquid behave as follows:
- Compatible and additive when the salt is present in excess in comparison to the ionic liquid
- Incompatible, but still thermo-stabilizing, when the ionic liquid is present in excess in comparison to the salt.

Figure 17 summarizes the results of the addition of a combination of cholineCl/sorbitol and lactobionic acid. These results are more complex than data of the combinations tested before. (embodiment not covered by the claimed invention)

Figure 17: Effect of different combinations of a hydrated deep eutectic mixture of choline chloride and sorbitol (molar ratio 5: 2) and lactobionic acid in a composition of the model protein lactate dehydrogenase. In blue the measured change in Tₘ of the combined formulation. Stacked in black and red are the changes is Tₘ determined for the individual excipients separately. One would have to conclude to a synergistic effect, if the blue bar would surpass the stacked bar of black and red.

Different interactions are observed depending on the concentration of the containing ionic liquid: If only a low concentration of the ionic liquid is used (0.5 M) and the addition of lactobionic acid is in a ratio of 2:1, a synergistic effect is received, and the model protein is stabilized more than by the individual components.

With a 1.5 molar ionic liquid, a similar effect is observed, however under these conditions a larger excess of the ionic liquid is required to produce a synergistic effect. When the excess of the ionic liquid is increased to 6-fold, a synergistic stabilization is found. At very high concentrations of the ionic liquid (2.5 M), only an effect of small concentrations of lactobionic acid can be probed for. Using a 10-fold excess of the ionic liquid over lactobionic acid leads to a synergistic effect of both. However, when only 0.15 M lactobionic acid (16: 1) is used, an additive effect is more likely to be observed.

For the interaction of an ionic liquid and a sugar acid in the model composition it is concluded that:
- The interaction of the ionic liquid and the sugar acid is concentration dependent
- The interaction of the ionic liquid and the sugar acid is also dependent on the ratio ionic liquid to sugar acid
- When a sufficiently large excess of ionic liquid is used, we observe a synergistic effect of both excipients on the thermostability of a protein
- The ratio ionic liquid:sugar acid that is required for such a synergistic effect increases with an increasing fraction of the ionic liquid
- The concentration of the sugar acid must be minimum 0.25 M in order to observe a synergistic effect of the two excipients

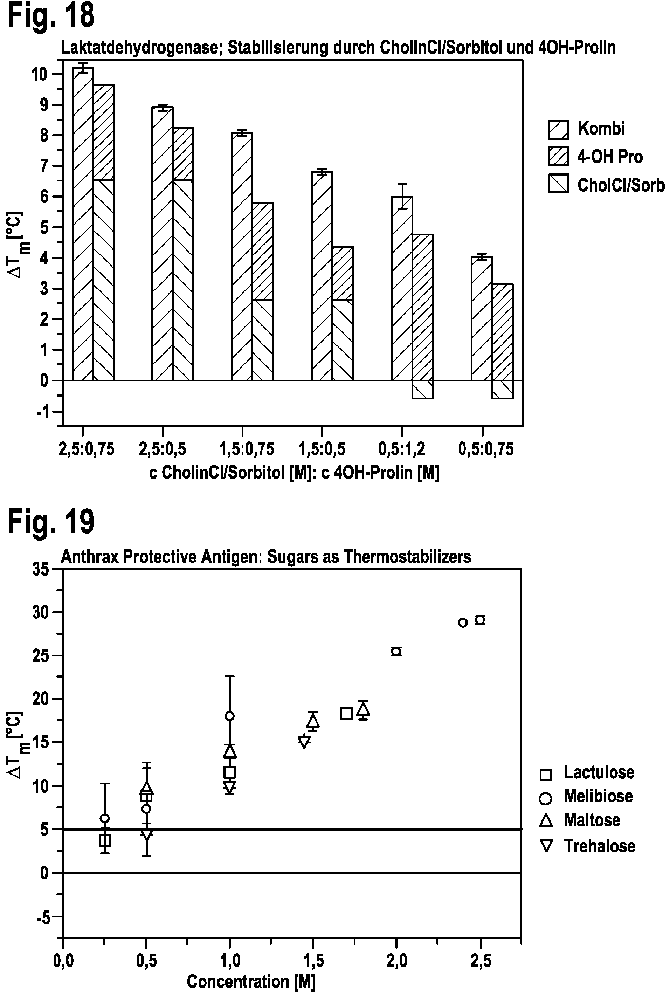

Figure 18 summarizes the results of combinations of Choline chloride/Sorbitol and 4-OH-Proline (embodiment not covered by the claimed invention)

Figure 18: Effect of different combinations of a hydrated deep eutectic mixture of Choline Chloride and Sorbitol (molar ratio 5: 2) and 4-hydroxyl-proline in the model protein composition comprising lactate dehydrogenase. In blue the measured change in Tₘ of the combined formulation. Stacked in black and red are the changes is Tₘ determined for the individual excipients separately. One would have to conclude to a synergistic effect, if the blue bar would surpass the stacked bar of black and red.

The measured results show that the combination of the ionic liquid with the amino acid over the entire ranges of concentrations and of ratios tested has a synergistic thermostabilizing effect on LDH, causing 5% to 36% higher thermal stability than the effect which was expected of the two single components.

A summary of the excipient combinations used, their concentration range and their effect on LDH can be found in Table 4 below.

**Table 4:**

| Excipient Combination | | Concentration range used | | Concentration range of thermostabilizer | | | | % of indivdual components | Comments |
|---|---|---|---|---|---|---|---|---|---|
| | | [M] | | [M] | | | | | |
| | | | | >0 °C | | min. 5 °C | | | |
| A | B | A | B | A | B | A | B | | |
| Potassium dihydrogen-phosphate | Lactobionic acid | 0.25-0.75 | 0.1 - 0.4 | 0.25-0.75 | 0.1 - 0.4 | 0.25-0.75 | 0.1-0.4 | 90 - 104 | additive |
| Potassium dihydrogen-phosphate | 4-hydroxyl proline | 0.5-1 | 0.15-0.9 | 0.5- 1 | 0.15-0.9 | 0.5- 1 | 0.15- 0.9 | 95 - 100 | additive |
| Potassium dihydrogen-phosphate | Cholinchloride Cholinchloride / sorbitol molar ratio 5: 2 | 0.5-1 | 0.05-0.5 | 0.5- 1 | 0.05-0.5 | 0.5- 1 | 0.05- 0.5 | 58 - 102 | non-additive |
| Cholinchloride Cholinchloride/ sorbitol molar ratio 5: 2 | Lactobionic acid | 0.1-0.5 | 0.15-0.4 | only with a clear excess of one component | | only with a clear excess of one component | | -173 - 130 | non-additive |
| Cholinchloride Cholinchloride/ sorbitol molar ratio 5: 2 | 4-hydroxyl proline | 0.1-0.5 | 0.5-1.2 | 0.1-0.5 | 0.5- 1.2 | 0.5-1.2 | 0.5- 1.2 | 105 - 136 | additive at times synergistic |

| | | | | | | | | | |
|---|---|---|---|---|---|---|---|---|---|
| *molarity based on amount of Choline chloride in resulting volume. | | | | | | | | | |

### Screening for thermostabilizing excipients using vaccine proteins

In the following chapter the screening results for excipient molecules using three fairly different vaccine proteins are discussed. Not all potential excipient molecules are tested on all vaccine proteins. The aim is to identify excipient molecules that can elevate the mean unfolding temperature of three very different proteins to temperatures above 60°C.

### Experimental approach

We employ fluorescence spectroscopy to determine the unfolding transition mid-point of our proteins. Specifically, we use Nano-Differential Scanning Fluorimetry allowing for a high throughput screening.

Proteins are used at a concentration of 0.1 mg/ml, which corresponds well to the protein concentration in marketed vaccine doses. Excipients are titrated up to concentrations close to their solubility limit. All excipients are prepared in the native formulation buffer of the vaccine protein, with the exception of choline dihydrogenphosphate. The latter is dissolved in water and the pH is adjusted to match the pH of the native vaccine protein formulation (details see in the following). In this case the dihydrogenphosphate ion will act as a buffering agent. A protein is mixed with an excipient and it is allowed to equilibrate at room temperature for 15 minutes.

### Screening for thermostabilizing excipients using Anthrax Protective Antigen

Anthrax Protective Antigen (AnthraxPA) is the main protein component of the Anthrax vaccine. Its native formulation is 5 mM HEPES with 50 mM NaCl at a pH of 7.5. The native mean unfolding temperature is 48°C. Three classes of excipients are used in the tests to stabilize AnthraxPA: Sugars, ionic liquids/salts, and osmolytes. The obtained results are compared with results using trehalose, which is a widely used excipient for various purposes.

Figure 19 shows the results for thermo-stabilizing AnthraxPA using sugars.

Figure 19: Effect of sugars as excipient candidates on the mean unfolding temperature of AnthraxPA. The black line indicates a dTm =0°C. The red line indicates dTₘ = 5°C. Molecules that are able to stabilize AnthraxPA by more than 5°C are subjected to a second screening with a different model protein.

In this first group lactulose (embodiment not covered by the claimed invention), melibiose (embodiment not covered by the claimed invention), and maltose are identified as good thermo-stabilizers and will be used for further assessment. Already at relatively low concentrations (250 mM) the required 5°C additional thermo-stability is observed. With almost 30°C, melibiose is clearly most effective in shifting the Tₘ of AnthraxPA to higher temperatures. All sugars tested in this group are more efficient thermo-stabilizers than trehalose.

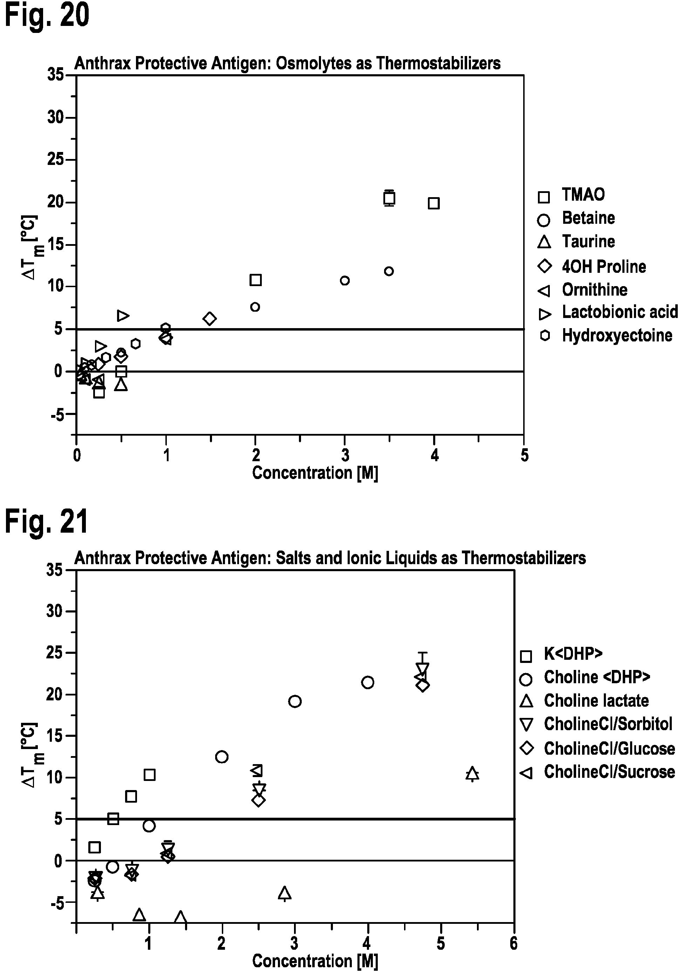

Figure 20 shows the results for thermo-stabilizing AnthraxPA using osmolytes (embodiment not covered by the claimed invention)

Figure 20: Effect of osmolytes as excipient candidates on the mean unfolding temperature of AnthraxPA. The black line indicates a dTₘ =0°C. The red line indicates dTₘ = 5°C. Molecules that are able to stabilize AnthraxPA by more than 5°C are subjected to a second screening with a different model protein.

In this second group are several known protein stabilizers, such as for example Taurine, which are unable to stabilize the vaccine protein used herein. Among the osmolytes it is found that TMAO, betaine, 4-hydroxyl-proline, hydroxyectoine, and lactobionic acid are according to the present definition good thermo-stabilizers and are therefore selected for further testing. However, only TMAO surpasses trehalose as a thermo-stabilizer.

Figure 21 shows the results for thermo-stabilizing AnthraxPA using salts and ionic liquids. (embodiment not covered by the claimed invention)

Figure 21: Effect of excipient candidates on the mean unfolding temperature of AnthraxPA. The black line indicates dTₘ =0°C. The red line indicates dTₘ = 5°C. Molecules that are able to stabilize AnthraxPA by more than 5°C are subjected to a second screening with a different model protein. In order to use the same type of plot for the hydrated deep eutectic liquids as for the other excipients, the molar concentration of choline chloride was estimated based on the weight of material used and the volume of the prepared liquid

In this third group of molecules it is found that according to the definition above potassium dihydrogenphosphate, choline dihydrogenphosphate, choline lactate, and hydrated deep eutectic mixtures of choline chloride with - sorbitol, -glucose, and -sucrose respectively under some conditions can be classified as good thermostabilizer.

### Screening for thermostabilizing excipients using Tetanus toxoid

Tetanus toxoid (T_{d}) is the inactivated form of Clostridium tetanii toxin and prepared by formaldehyde cross linking of the toxin monomers. The use of formaldehyde cross-linking generates are large protein complex, which is no longer toxic, but still presents the antigenic structures to be recognized by the immune system. Its native formulation is 100 mM histidine with 100 mM NaCl at a pH of 7,0. The native mean unfolding temperature is 67°C.

Three classes of excipients are used in the efforts to stabilize T_{d}: sugars, ionic liquids/salts, and osmolytes are compared with the obtained results of trehalose, which is a widely used excipient for various purposes.

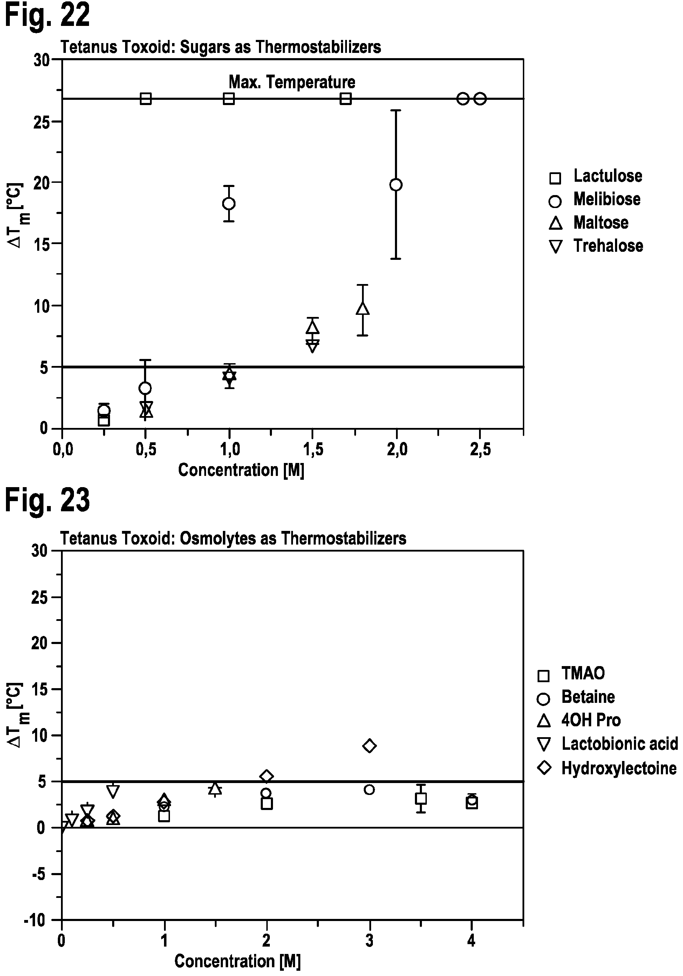

Figure 22 shows the results for thermo-stabilizing T_{d} using sugars.

Figure 22: Effect of sugars as excipient candidates on the mean unfolding temperature of T_{d}. The black line indicates a dTₘ =0°C. The red line indicates dTₘ = 5°C. Molecules that are able to stabilize T_{d} by more than 5°C are subjected to a second screening with a different model protein.

In this first group lactulose (embodiment not covered by the claimed invention), melibiose (embodiment not covered by the claimed invention), and maltose are identified as good thermo-stabilizers. The max. temperature line indicates that for the affected excipient concentration no complete unfolding transition could be observed, since the equipment could not heat the samples any further and the unfolding transition was incomplete. Like in the previously discussed experiments on AnthraxPA, lactulose, melibiose, and maltose are more efficient thermo-stabilizers than trehalose (embodiment not covered by the claimed invention).

Figure 23 shows the results for thermo-stabilizing T_{d} using osmolytes (embodiment not covered by the claimed invention).

Figure 23: Effect of osmolytes as excipient candidates on the mean unfolding temperature of T_{d}. The black line indicates a dTm =0°C. The red line indicates dTₘ = 5°C. Molecules that are able to stabilize T_{d} by more than 5°C are subjected to a second screening with a different model protein.

In this second group of excipients we observe that only hydroxyectoine can be identified as a good thermostabilizer. It is also the only substance tested in this group that performs better than trehalose.

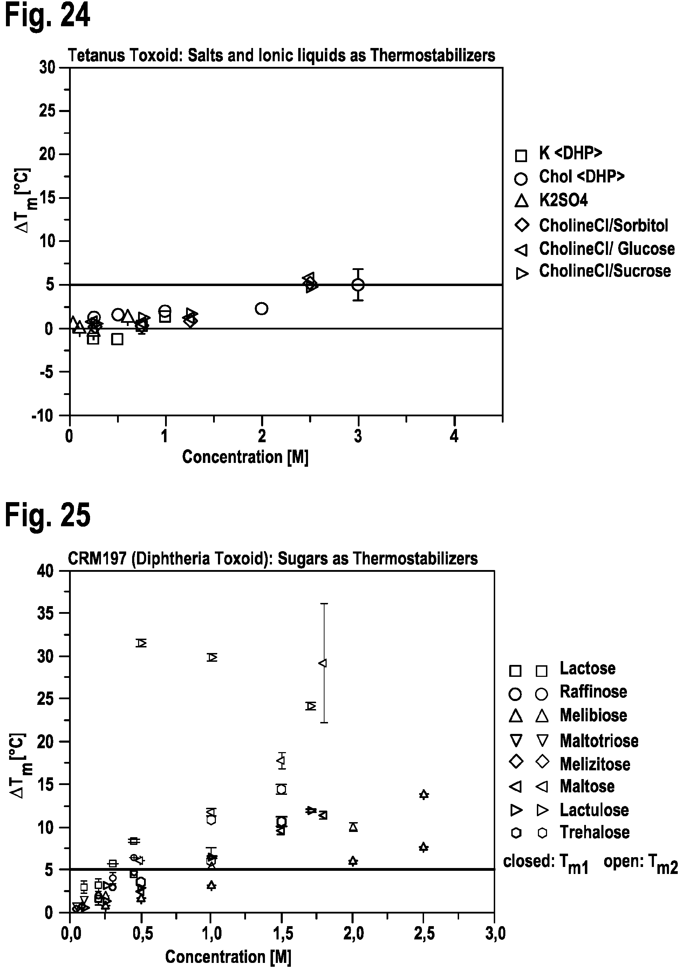

Figure 24 shows the results for thermo-stabilizing T_{d} using ionic liquids (embodiment not covered by the claimed invention).

Figure 24: Effect of excipient candidates on the mean unfolding temperature of T_{d}. The black line indicates dTₘ =0°C. The red line indicates dTₘ = 5°C. Molecules that are able to stabilize T_{d} by more than 5°C are subjected to a second screening with a different model protein. In order to use the same type of plot for the hydrated deep eutectic liquids as for the other excipients, the molar concentration of choline chloride was estimated based on the weight of material used and the volume of the prepared liquid.

In this third group of excipients it is observed that Choline dihydrogenphosphate is the most efficient thermostabilizer in this group and also more efficient than trehalose. Hydrated deep eutectic mixtures of choline chloride with -sorbitol, -glucose, and -sucrose respectively, can be classified as good thermostabilizers.

### Screening for thermostabilizing excipients using Diphteria toxoid CRM197

Diphteria toxoid (CRM197) is the inactivated form of Corynebacterium diphteria toxin. The introduction of a point mutation renders the toxin harmless, but still presents the antigenic structures to be recognized by the immune system. Its native formulation is 20 mM HEPES with 150 mM NaCl at a pH of 8,0. The teritary structure unfolding, which is the type of unfolding monitored using fluorescence spectroscopy, of CRM197 is rather complex: It unfolds in a biphasic transition with the first transition representing a local structural change, while the second transition represents the global unfolding of the protein. Since neither temperature can be linked specifically to the ability of CRM197 to elicit immunity, both transitions are analyzed with respect to the effectivity of our excipients. Also, here three classes of excipients are used: Sugars, ionic liquids/salts, and osmolytes and compared with results obtained with trehalose, which is a widely used excipient for various purposes.

Figure 25 shows the results for thermo-stabilizing CRM using sugars.

Figure 25: Effect of sugars as excipient candidates on the mean unfolding temperature of CRM. The red line indicates dTₘ = 5°C. Molecules that are able to stabilize CRM by more than 5°C locally (closed symbols) and globally (open symbols) are considered as excipient candidates and, if required, screened with different vaccine proteins.

In this first group lactulose (embodiment not covered by the claimed invention), melibiose (embodiment not covered by the claimed invention), and maltose are identified as being good thermo-stabilizers performing better than trehalose (embodiment not covered by the claimed invention).

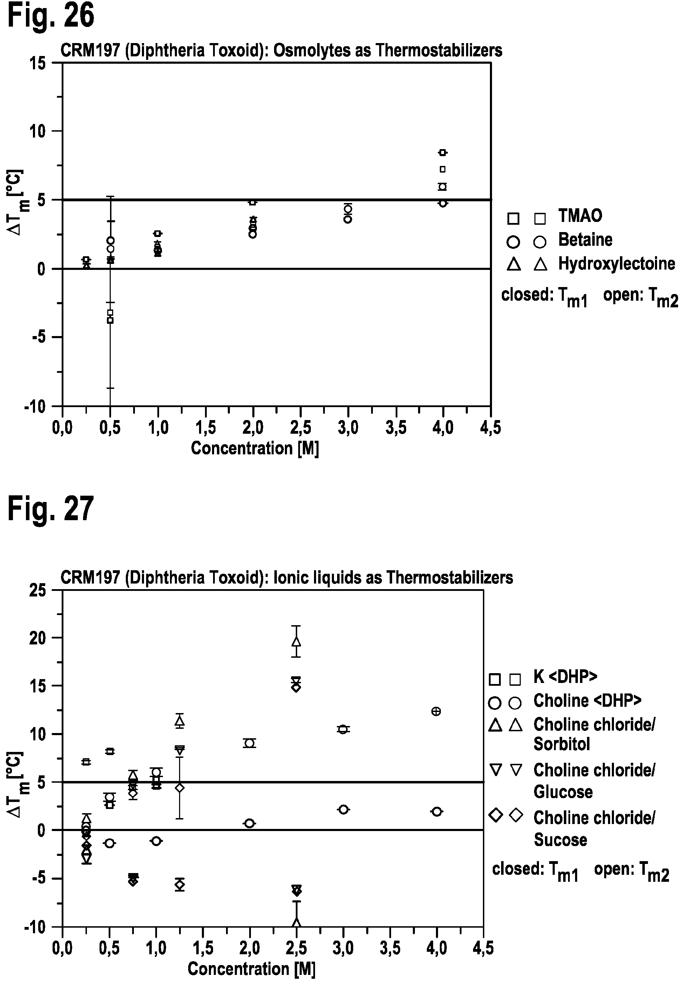

Figure 26 shows the results for thermo-stabilizing CRM using osmolytes (embodiment not covered by the claimed invention).

Figure 26: Effect of osmolytes as excipient candidates on the mean unfolding temperature of CRM. The black line indicates a dTₘ = 0°C. The red line indicates dTₘ = 5°C. Molecules that are able to stabilize CRM by more than 5°C locally (closed symbols) and globally (open smybols) are considered as excipient candidates.

In this second group of excipients it is observed that only TMAO (embodiment not covered by the claimed invention) can be identified as a good thermo-stabilizer.

Figure 27 shows the results for thermo-stabilizing CRM using ionic liquids (embodiment not covered by the claimed invention).

Figure 27: Effect of excipient candidates on the mean unfolding temperature of CRM. The black line indicates dTₘ =0°C. The red line indicates dTₘ = 5°C. Molecules that are able to stabilize CRM by more than 5°C are subjected to a second screening with a different model protein. In order to use the same type of plot for the hydrated deep eutectic liquids as for the other excipients, the molar concentration of choline chloride was estimated based on the weight of material used and the volume of the prepared liquid

In this third group of excipients it is observed that choline dihydrogenphosphate can be classified as good thermo-stabilizers although the local unfolding transition (Tm1) is not stabilized much. Besides choline dihydrogenphosphate, all other choline based ionic liquids thermo-stabilize CRM more efficient than trehalose. However, the choline chloride based ionic liquids have a substantially destabilizing effect on the first unfolding transition of CRM. This is probably caused by the high amount of charges present in these ionic liquids.

### Assessment of excipient effect on the colloidal stability of vaccine proteins

After five molecules have been identified as potential excipients to elicit stability at high temperatures to proteins, these molecules are subjected to a more detailed evaluation. A key aspect therein is to determine if the selected excipient has any effect on the colloidal stability of a protein. In other words: It is needed to determine if a protein shows an altered tendency to aggregate in the presence of the excipient. If aggregation is prevented, the respective excipient will show an additional benefit.

In order to assess the aggregation behavior of a protein, dynamic light scattering is used. The hydrodynamic radius of the protein is measured and this value can be used to assess if the protein is monomeric or has an aggregated state. The five selected excipients are added to a solution comprising the vaccine protein in a concentration of 0.1 mg/ml. As in the previous section, each vaccine protein is formulated in its native formulation and each excipient is prepared in the corresponding native formulation of the vaccine protein. Also, in this set of experiments choline dihydrogenphosphate is prepared without any buffer components, but the pH of the excipient stock solution is adjusted to match the pH of the vaccine protein formulation.

Vaccine protein and excipient are mixed together generating a test formulation with 0.1 mg/ml vaccine protein content and the desired concentration of the excipient (details are given below).

To assess the colloidal stability of the vaccine proteins at elevated temperatures, the samples are loaded into a 316-well plate, which is placed into the Dynapro Plate Reader III (Wyatt Technology). The hydrodynamic radius Rₕ of the vaccine protein is determined at room temperature. Then the plate is heated for 5 minutes to 40°C and then cooled back to room temperature, where Rₕ is determined again. In this way different temperatures in the range of 40-80°C is assayed in 10°C increments.

Figure 28 shows the results for AnthraxPA as an example (embodiment not covered by the claimed invention).

Figure 28: The hydrodynamic radius of AnthraxPA after a 5 minutes incubation at elevated temperatures.

For the natively formulated AnthraxPA a low hydrodynamic radius is observed at room temperature. This low Rₕ does not appear to be affected by a 5 minutes incubation at 40°C. However, after a subsequent 5 minutes at 50°C a dramatic increase in Rₕ is observed, indicating that at 50°C AnthraxPA is aggregated. This temperature is called T_{agg}. From this it is concluded that for natively formulated AnthraxPA the aggregation temperature T_{agg} is 50°C. Addition of various excipients elevates T_{agg} to temperatures of up to 80°C.

To analyze if the observed change in T_{agg} is in fact the suppression of aggregation or if it is a mere consequence of a higher conformational stability of the protein T_{agg} is plotted vs. Tₘ, which is shown in Figure 29.

Figure 29: T_{agg} vs. Tₘ. Since it is probed for T_{agg} using a 10°C increment, a 10°C interval around Tₘ is defined where an aggregation is considered to strictly accompany unfolding (green area).

If T_{agg} is elevated beyond that regime, the excipient is considered to suppress aggregation (yellow area). If T_{agg} is reduced below that regime, the excipient would be considered to promote aggregation (red area).

It is observed that melibiose (embodiment not covered by the claimed invention) suppresses aggregation of CRM (2M and above) and Tetanus

toxoid (2.4M). Since the latter does not aggregate there are no corresponding data points in Figure 29. Furthermore, maltose is suppressing the aggregation of CRM (1,5M and above), which also yields to a complete lack of data points in Figure 21.

### Assessment of excipient effect on the secondary structure stability of vaccine proteins

In addition to probing the tertiary structure of the vaccine proteins, the five excipients are subjected to an analysis method reporting on their effect on the secondary structure content of the vaccine proteins.

One concentration of each excipient is analyzed with all three vaccine proteins. The protein concentration we use is 0.5 mg/ml. Furthermore, it is necessary to use a different buffer system than in the previous experiments in order not to interfere with light detection in this setup. However, it can be shown that this change in buffer does not affect the stability of the proteins used in this study.

The vaccine proteins are formulated in a buffer comprising of 5 mM sodium dihydrogen phosphate and sodium chloride (50-150 mM) maintaining the same pH-level as in the respective native formulations. Spectra are recorded from 280-200 nm (in some cases 210 nm) with a data pitch of 1 nm, slit width of 100 µm, response 2 s, a speed of 100 nm/s and 1 mm optical path length. Three spectra are averaged for each temperature.

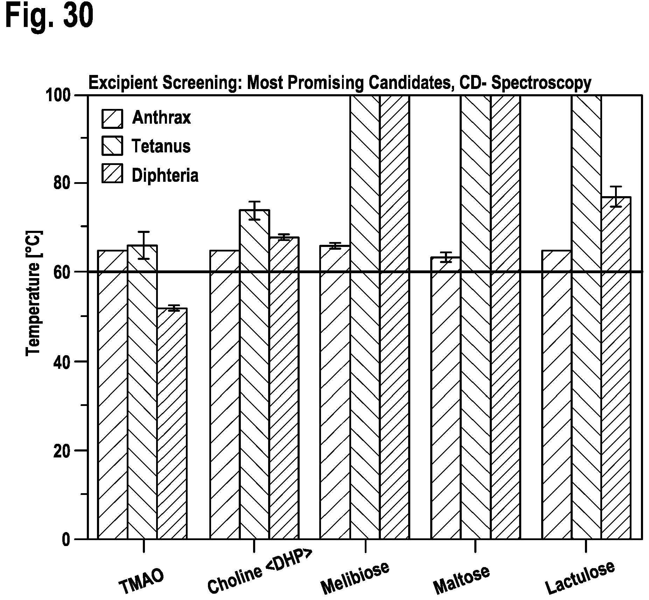

Figure 30 shows the determined transition temperatures.

Figure 30 Transition temperatures of AnthraxPA, Tetanus and Diphtheria toxoid in formulations containing newly identified thermo-stabilizers

Before summarizing the results, there are two observations to be noted, that are important to put the data in the appropriate context:
Firstly, for AnthraxPA two heat induced transitions are observed. Without prior information on how each transition affects the ability of AnthraxPA and affects the immunogenicity of the protein, the lower transition temperature is used for analysis. In a formulation with lactulose (embodiment not covered by the claimed invention), the two unfolding transitions occur in a rather narrow temperature regime and neither can be determined with the required accuracy. Therefore, the highest temperature is reported, where no structural change has occurred. Overall, the unfolding temperatures of Anthrax should be regarded as a lower limit rather than a transition mid-point.
Secondly, there are conditions, where no change in secondary structure is observed, even at very high temperatures. However, the information from DLS and fluorescence measurements is required to properly assess the overall stability and mode of action of the excipients.

**Table 5 summarizes the results of the experiments:**

| Excipient | Concentration [M] | CD spectroscopy | | |
|---|---|---|---|---|
| | | Transition temperature [°C] | | |
| | | AnthraxPA | Td | CRM197 |
| None | - | 59 | No unfolding | No unfolding |
| Lactulose | 1,7 | 65 | No unfolding | 77 |
| Melibiose | 2,4 | 66 | No unfolding | No unfolding |
| Maltose | 1,8 | 63 | No unfolding | No unfolding |
| Choline dihydrogen phosphate | 3,5 | 65 | 74 | 68 |
| TMAO | 3,5 | 65 | 66 | 52 |

### Effect of five selected excipients on vaccine protein stability

The effect of excipients selected from three different substance groups on the conformational and colloidal stability of vaccine proteins is determined. For the conformational stability it is possible to differentiate between effects on the secondary and tertiary structure.

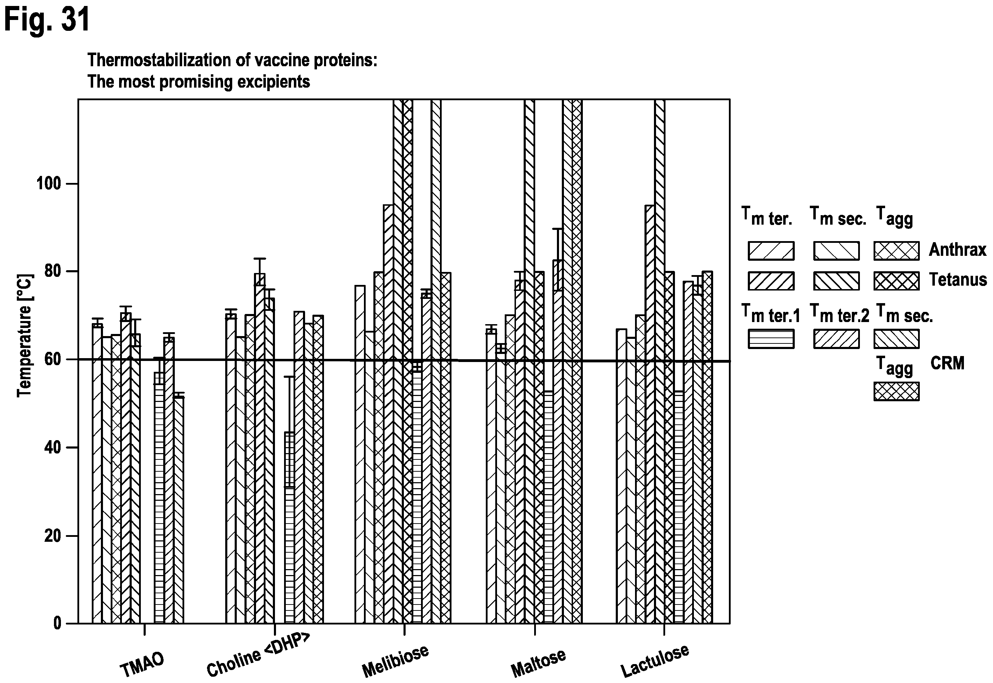

Figure 31 gives a summary of all results for each protein and excipient combination.

Figure 31: Summary of fluorescence, CD and DLS data obtained for AnthraxPA, CRM197 and Tetanus toxoid. The black line at 60°C represents the desired stability target. With the information at hand, the effect of each excipient on each protein can be explained.
i) TMAO (embodiment not covered by the claimed invention): Among the set of excipients used TMAO appears to be the weakest of the very good thermo-stabilizer.
ii) choline dihydrogenphosphate (embodiment not covered by the claimed invention): This ionic liquid is a stronger thermo-stabilizer than TMAO and stabilizes CRM better than TMAO.

i) Melibiose (embodiment not covered by the claimed invention): The sugars tested herein generally show the best thermo-stabilizing. melibiose is a more efficient thermo-stabilizer than trehalose for all three vaccine proteins tested. Melibiose suppresses aggregation for Tetanus and CRM.
ii) Maltose: Its thermo-stabilizing effect is similar to melibiose. Maltose is stabilizing all vaccine proteins significantly above 60°C, but Melibiose seems to have a stronger stabilizing effect. Maltose is a more efficient thermo-stabilizer than trehalose for all three vaccine proteins tested. On the other hand, Maltose is used at a much lower concentration making this excipient much easier to use. Maltose suppresses aggregation of CRM, and secondary structure unfolding of CRM and Td.
iii) Lactulose (embodiment not covered by the claimed invention): Similar to the sugars mentioned before, Lactulose stabilizes all vaccine proteins to temperatures above 60°C. However, in contrast to the other sugars, a secondary structure unfolding of CRM is observed and no aggregation prevention. But Lactulose shows a similar effectiveness as a thermo-stabilizer as maltose and is more efficient than trehalose for all three vaccine proteins. Both sugars are used in similar concentrations (1.7 M Lactulose and 1.8 M Maltose), whereas melibiose is effective if a higher concentrated solution is applied (2.4M).

## Claims

1. A method for increasing the thermostability of a liquid protein or peptide formulation, comprising the step of combining a protein or peptide solution with maltose in a concentration in the range of 0.5 - 1.8 M, wherein the protein or peptide stays in its native, not unfolded form during storage at temperatures higher than 50 °C and up to 60 °C, thus obtaining a stabilized formulation, the increased thermostability being measured as the change in the transition mid-point Tₘ determined by subtracting T_{m native} from T_{m excipient} , each Tₘ being measured by Nano-Differential Scanning Fluorimetry (nanoDSF) using a protein solution comprising the protein in a concentration of 0.05-1,8 mg/ml heated with a heating rate of 1°C/minute.

2. The method according to claim 1, wherein a protein or peptide formulation containing the protein or peptide in a concentration of 0.05 to 2 mg/ml is stabilized.

3. The method according to any one of the claims 1 to 2, wherein a protein or peptide formulation is stabilized and wherein the protein or peptide is selected from the group consisting of viral protein subunits, vaccines comprising viral protein subunits, enzymes, nanobodies and monobodies.

4. The method according to any one of the claims 1 to 2, wherein a protein or peptide formulation is stabilized and wherein the protein or peptide is selected from the group consisting of viruses, attenuated viruses, viral-like particles, viral-derived proteins, viral protein subunits, and vaccines comprising viral protein subunits.

5. The method according to any one of the claims 1 to 2, wherein a protein or peptide formulation of vaccines comprising viral protein subunits is stabilized.

6. The method according to any one of the claims 1 to 2, wherein a protein or peptide formulation comprising an enzyme is stabilized.

7. The A method of any one of the claims 1 to 6, wherein the pH value of the formulation is adjusted to pH 6 - 8, preferably to 7 to 7.5.

8. The method of any one of the claims 1 to 7, wherein the pH value of the formulation is controlled by the addition of a buffer selected from the group potassium phosphate, sodium phosphate, sodium acetate, histidine, imidazole, sodium citrate, sodium succinate, HEPES, Tris, Bis-Tris and ammonium bicarbonate.

9. The A method of any one of the claims 1 to 7, wherein the formulation comprises polymers to stabilize the protein in an amount of at least 0,1% w/v and/or surfactants in an amount of at least 0,005 % w/v.

10. The method of any one of the claims 1 to 7, wherein the formulation comprises divalent cations in a concentration in the range from 0,1 to 100 mM and amino acids in a concentration in the range from about 0,1 to 1 % w/v to stabilize the comprising protein and to adjust pH and osmolarity of the solution.

## Patentansprüche

1. Verfahren zur Erhöhung der Thermostabilität einer flüssigen Protein- oder Peptidformulierung, umfassend den Schritt des Kombinierens einer Protein- oder Peptidlösung mit Maltose in einer Konzentration im Bereich von 0,5 - 1,8 M, wobei das Protein oder Peptid während einer Lagerung bei Temperaturen von mehr als 50°C und bis zu 60°C in seiner nativen, nicht entfalteten Form bleibt, wodurch eine stabilisierte Formulierung erhalten wird, wobei die erhöhte Thermostabilität als die Änderung des Übergangsmittelpunkts Tₘ gemessen wird, der bestimmt wird, indem T_{m nativ} von T_{m Trägerstoff} subtrahiert wird, wobei der Tₘ jeweils durch Nano-Differenzscan-Fluorimetrie (nanoDSF) unter Verwendung einer Proteinlösung, die das Protein in einer Konzentration von 0,05-1,8 mg/ml enthält und mit einer Heizrate von 1°C/Minute erhitzt wird, gemessen wird.

2. Verfahren nach Anspruch 1, wobei eine Protein- oder Peptidformulierung, die das Protein oder Peptid in einer Konzentration von 0,05 bis 2 mg/ml enthält, stabilisiert wird.

3. Verfahren nach irgendeinem der Ansprüche 1 bis 2, wobei eine Protein- oder Peptidformulierung stabilisiert wird und wobei das Protein oder Peptid ausgewählt ist aus der Gruppe bestehend aus viralen Proteinuntereinheiten, Impfstoffen, die virale Proteinuntereinheiten enthalten, Enzymen, Nanoantikörpern und Monoantikörpern.

4. Verfahren nach irgendeinem der Ansprüche 1 bis 2, wobei eine Protein- oder Peptidformulierung stabilisiert wird und wobei das Protein oder Peptid ausgewählt ist aus der Gruppe bestehend aus Viren, abgeschwächten Viren, virusähnlichen Partikeln, von Viren stammenden Proteinen, viralen Proteinuntereinheiten und Impfstoffen, die virale Proteinuntereinheiten enthalten.

5. Verfahren nach irgendeinem der Ansprüche 1 bis 2, wobei eine Protein- oder Peptidformulierung von Impfstoffen, die virale Proteinuntereinheiten enthalten, stabilisiert wird.

6. Verfahren nach irgendeinem der Ansprüche 1 bis 2, wobei eine Protein- oder Peptidformulierung, die ein Enzym enthält, stabilisiert wird.

7. Verfahren nach irgendeinem der Ansprüche 1 bis 6, wobei der pH-Wert der Formulierung auf pH 6 - 8, vorzugsweise auf 7 bis 7,5, eingestellt wird.

8. Verfahren nach irgendeinem der Ansprüche 1 bis 7, wobei der pH-Wert der Formulierung durch Zugabe eines Puffers, der ausgewählt ist aus der Gruppe Kaliumphosphat, Natriumphosphat, Natriumacetat, Histidin, Imidazol, Natriumcitrat, Natriumsuccinat, HEPES, Tris, Bis-Tris und Ammoniumhydrogencarbonat, gesteuert wird.

9. Verfahren nach irgendeinem der Ansprüche 1 bis 7, wobei die Formulierung Polymere zur Stabilisierung des Proteins in einer Menge von mindestens 0,1 % w/v und/oder Tenside in einer Menge von mindestens 0,005 % w/v enthält.

10. Verfahren nach irgendeinem der Ansprüche 1 bis 7, wobei die Formulierung zweiwertige Kationen in einer Konzentration im Bereich von 0,1 bis 100 mM und Aminosäuren in einer Konzentration im Bereich von etwa 0,1 bis 1 % w/v zur Stabilisierung des enthaltenen Proteins und zur Einstellung des pH-Werts und der Osmolarität der Lösung enthält.

## Revendications

1. **Méthode** destinée à augmenter la thermostabilité d'une formulation liquide de protéine ou de peptide, comprenant l'étape consistant à associer une solution de protéine ou de peptide avec du maltose selon une concentration dans la plage de 0,5 - 1,8 M, où la protéine ou le peptide reste dans sa forme native, non repliée, lors d'un stockage à des températures supérieures à 50°C et allant jusqu'à 60°C, conduisant ainsi à une formulation stabilisée, la thermostabilité accrue étant mesurée en tant que changement de la transition au point milieu Tₘ déterminé en soustrayant la T_{m native} de la T_{m excipient}, chaque Tₘ étant mesurée par Fluorimétrie à Balayage Nano-Différentiel (nanoDSF) en utilisant une solution de protéine comprenant la protéine selon une concentration de 0,05-1,8 mg/ml chauffée selon une vitesse de chauffage de 1°C/minute.

2. Méthode selon la revendication 1, dans laquelle une formulation de protéine ou de peptide contenant la protéine ou le peptide selon une concentration allant de 0,05 à 2 mg/ml est stabilisée.

3. Méthode selon l'une quelconque des revendications 1 à 2, dans laquelle une formulation de protéine ou de peptide est stabilisée et où la protéine ou le peptide est choisi(e) dans le groupe constitué par les sous-unités de protéines virales, les vaccins comprenant des sous-unités de protéines virales, les enzymes, les nano-anticorps et les mono-anticorps.

4. Méthode selon l'une quelconque des revendications 1 à 2, dans laquelle une formulation de protéine ou de peptide est stabilisée et où la protéine ou le peptide est choisi(e) dans le groupe constitué par les virus, les virus atténués, les particules pseudo-virales, les protéines dérivées de virus, les sous-unités de protéines virales, et les vaccins comprenant des sous-unités de protéines virales.

5. Méthode selon l'une quelconque des revendications 1 à 2, dans laquelle une formulation de protéine ou de peptide de vaccins comprenant des sous-unités de protéines virales est stabilisée.

6. Méthode selon l'une quelconque des revendications 1 à 2, dans laquelle une formulation de protéine ou de peptide comprenant une enzyme est stabilisée.

7. Méthode selon l'une quelconque des revendications 1 à 6, dans laquelle la valeur de pH de la formulation est ajustée à pH 6 - 8, préférablement de 7 à 7,5.

8. Méthode selon l'une quelconque des revendications 1 à 7, dans laquelle la valeur de pH de la formulation est contrôlée par l'addition d'un tampon choisi dans le groupe constitué par le phosphate de potassium, le phosphate de sodium, l'acétate de sodium, l'histidine, un imidazole, le citrate de sodium, le succinate de sodium, l'HEPES, le Tris, le Bis-Tris et le bicarbonate d'ammonium.

9. Méthode selon l'une quelconque des revendications 1 à 7, dans laquelle la formulation comprend des polymères destinés à stabiliser la protéine selon une quantité d'au moins 0,1% p/v et/ou des agent tensioactifs selon une quantité d'au moins 0,005% p/v.

10. Méthode selon l'une quelconque des revendications 1 à 7, dans laquelle la formulation comprend des cations divalents selon une concentration dans la plage allant de 0,1 à 100 mM et des acides aminés selon une concentration dans la plage allant d'environ 0,1 à 1% p/v afin de stabiliser la protéine et d'ajuster le pH et l'osmolarité de la solution.
